# EUROPEAN PATENT SPECIFICATION

(11) **EP 3 843 649 B1**
(45) Date of publication and mention of the grant of the patent: **04.06.2025**
(21) Application number: 19797785.3
(22) Date of filing: 27.08.2019
(51) Int. Cl.: A61B 18/14, A61B 18/00

(54) **FLEXIBLE BIPOLAR ELECTRODE FOR TREATING MEDICAL CONDITIONS INCLUDING OBESITY AND CONSTIPATION**
FLEXIBLE BIPOLARE ELEKTRODE ZUR BEHANDLUNG VON KRANKHEITEN WIE FETTLEIBIGKEIT UND VERSTOPFUNG
ÉLECTRODE BIPOLAIRE FLEXIBLE POUR TRAITER DES ÉTATS MÉDICAUX NOTAMMENT L'OBÉSITÉ ET LA CONSTIPATION

(30) Priority: 28.08.2018 US 201862723927 P; 22.10.2018 US 201862748873 P
(43) Date of publication of application: 07.07.2021
(73) Proprietor: NewUro, B.V., 1016 PJ Amsterdam (NL)
(72) Inventor: JACKSON, Jerome, Los Altos, CA 94024 (US); STERN, Roger, A., Cupertino, CA 95014 (US); WANG, Benjamin, San Leandro, CA 94577 (US); AVERNI, Itzhak, Moshav Udim, Udim (IL); GANZ, Robert, Minnetonka, MN 55305 (US); BEN-EZRA, Omry, 6946215 Tel Aviv (IL)
(74) Representative: White, Andrew John
(86) International application number: PCT/IB2019/000969
(87) International publication number: WO 2020/044122

(56) References cited:
- US-A- 5 125 928
- US-A1- 2004 186 467
- US-A1- 2006 293 646

## Description

### BACKGROUND OF THE INVENTION

In bipolar energy delivery, electrodes of opposite polarities, typically adjacent to each other, may be brought in contact with a target tissue or material (the terms target tissue or target material may be used herein interchangeably and when one is used it should be taken as applying to the other as well). Alternating Current (AC) Radiofrequency (RF), Direct Current (DC), or microwave energy may be delivered between the two poles, through the tissue or material, causing tissue effects. These effects may be minimal, transient changes in (e.g. cellular membrane potential modification as in nerve or cardiac stimulation), or warming accompanied by permanent tissue changes - ranging from protein coagulation, through triggering of cellular apoptosis mechanisms followed by scarring, to complete tissue fulguration and evaporation.

Advantages of using bipolar electrodes may include a relatively local / shallow and therefore well controlled effect, as well as avoidance of the need for using a dispersive "ground" or "patient" electrode, as is the case with monopolar ablation.

In some applications, there may be a need to deliver the energy in an elongate pattern, such as a straight, circular, ellipsoid, curvilinear, zigzag, or any other substantially continuous linear or non-focal pattern.

This may for example be achieved using two parallel elongate electrodes. Such electrodes may for example be manufactured as part of a PCB (Printed Circuit Board). Specialized types of PCBs such as flexcircuits allow relatively good flexibility in the direction perpendicular to the plain of the PCB.

However, in some situations, for example when tight constraints of shape and/or dimensions must be met, the flexibility required of the linear electrodes may be greater than what may be possible using such a PCB. Furthermore, flexibility may be required in the direction parallel to the PCB (i.e. within the plane of the PCB), while PCBs, including flexible PCBs, may be extremely inflexible in this direction.

In such situations, the most appropriate mechanical properties may be similar to those of a flexible electrical wire. Unfortunately, a wire does not lend itself to bipolar use, as it is typically a single continuous conductor.

Two parallel wires could theoretically be used, although they are continuously insulated and would be difficult to make into bipolar pairs, especially if using a bifilar wire. In addition, using two parallel wires as a bipolar pair may require ensuring a constant distance between the wires, for example by placing spacers between them, gluing them to each other, or some other method. Even so, the resulting parallel track will only work well if both wires are placed in good contact with the target tissue along the desired path, which may be difficult to achieve. This is because it may be easy for such a design to twist, in which case there may be a significant difference between the poles in the size of surface area contacting the tissue. This may result with undesirable outcomes, such as reduced, or increased, energy delivery, leading to missing or excessive lesions in the target tissue or material.

Thus, there is a need to provide an electrode that can easily be used for bipolar energy delivery, is flexible, strong, reliable, maintains good contact of both polarities with the tissue regardless of its exact orientation and at the same time is easy to manufacture at low cost. Such an electrode should be capable of delivering the energy focally, in an elongate pattern, or in a planar pattern (over surfaces/areas).

Of note, another important distinct feature of the current invention is the high flexibility, or deformability, of the electrode. In other words, the electrode of the invention may be as thin and as flexible as a simple electrical wire, and as such, may be easily inserted into small lumens, folded and straightened, deployed and deformed to fit any required anatomy.

The mechanical activity of the stomach plays a major role in the digestive process in humans, both in health and in disease states. Modifying this activity may be an effective way of
treating gastrointestinal disease. The following brief anatomical, histological, and physiological background will make the concept clearer.

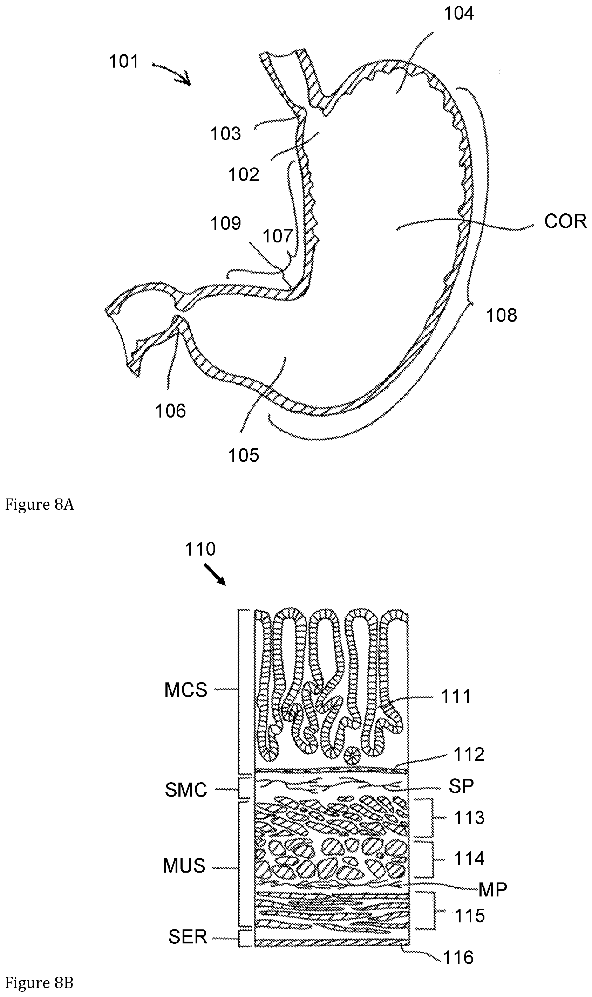

The gross anatomy of the normal human stomach is described in FIG. 8A which is a typical anterior view of a stomach 101. The cardia 102 is a 1-2 cm segment distal to the esophagogastric junction 103. The fundus 104 refers to the superior portion of the stomach that lies above an imaginary horizontal plane that passes through the esophagogastric junction. The antrum 105 is the smaller distal, one-fourth to one-third of the stomach. The narrow, 1-2-cm channel that connects the stomach and duodenum is the pylorus 106. The lesser curve 107 refers to the medial shorter border of the stomach, whereas the opposite surface is the greater curve 108. The angularis 109 is along the lesser curve of the stomach where the body and antrum meet. This junction is accentuated during peristalsis. The main body, or corpus COR of the stomach, extends between fundus 104 and antrum 105.

The human gastric wall comprises four main layers as illustrated in FIG. 8B. The innermost layer is the mucosa (MCS), followed by the submucosa (SMC), muscularis (MUS) and serosa (SER).

More particularly, FIG. 8B is a schematic illustration of a cross section through the gastric wall 110, depicting these layers in more detail.

Shown in FIG. 8B from top to bottom are: Mucosa MCS with gastric glands 111 and muscularis mucosae 112, which is a thin layer of smooth muscle within the mucosa. Submucosa SMC mainly comprises loose connective tissue and blood vessels. Muscularis MUS comprises three layers of smooth muscle: the innermost oblique 113 layer (unique to the stomach), the middle circular 114 layer and outermost longitudinal 115 layer. Serosa SER comprises loose connective tissue and blood vessels, and the visceral peritoneum layer 116.

The enteric nervous system (ENS) is one of the main divisions of the autonomic nervous system and comprises a mesh-like network of neurons that governs the function of the gastrointestinal tract.

The neurons of the ENS form two plexuses: the myenteric (Auerbach's) plexus (MP) between the inner and outer layers of the muscularis and the submucosal (Meissner's) plexus (SP) located in the submucosa.

Interstitial cells of Cajal are specialized cells which also form networks that are widely distributed within the submucosal, intra-muscular and inter-muscular layers of the gastrointestinal tract.

Physiological emptying of the stomach results from a grinding and pumping action comprising rings of circular muscle contractions that propagate from the main body (corpus) to the antrum, pushing digesta into the duodenum. This process has been described as gastric peristalsis. *(*All together now: from pacemakers to gastric peristalsis. Costa M., J Physiol. 2006 Feb 15; 571(Pt 1): 1.)

This peristalsis is initiated by a "pacemaker" which was localized to an area in the midcorpus along the greater curve, with potentials generated regularly at a mean frequency of about 3 cycles/min and propagated, increasing in amplitude and velocity as they approach the pylorus.

According to the current understanding of gastric physiology, contractions in the stomach are myogenic (i.e. are independent of neural activity). The origin of the myogenic activity in the stomach was tracked to the Interstitial Cells of Cajal (ICCs), with those ICCs in the Myenteric plexus responsible for slow longitudinal propagation, and those within the circular muscle layer responsible for fast "ring" contractions.

The relationship between gastric emptying and obesity is very complex. Some studies suggest a more rapid gastric emptying in obesity, although the opposite has been reported in some experimental settings. *(*Gastrointestinal motility in obesity. Wisen et al., J Intern Med. 1995 Apr;237(4):411-8*)*

The above gives rise to the idea that disrupting the interstitial cells network may impede gastric motility, thereby promoting satiation and satiety and slowing gastric emptying, and therefore may be a potential treatment for obesity.

Backing for this notion is found in a classic study, in which extracellular measurements of electrical activity done on intact stomachs of living humans, showed that isolation of the distal stomach from the natural pacemaker (due to complete transection of the gastric corpus) resulted in the appearance of a new pacemaker with a slower frequency. (Human gastric pacesetter potential. Site of origin, spread, and response to gastric transection and proximal gastric vagotomy. Hinder et al. Am J Surg. 1977 Jan;133(1):29-33.)

Based on the above concept, injections of botox into the gastric mucosa at the area of the antrum have shown efficacy in weight reduction, both in rats (The Effect of Intragastric Administration of Botulinum Toxin Type A on Reducing Adiposity in a Rat Model of Obesity Using Micro-CT and Histological Examinations . Park et al., Gut Liver. 2017 Nov; 11(6): 798-806*),* and in humans (Intragastric injection of botulinum toxin. A real alternative for obesity treatment? A systematic review. Sanchez et al., Nutr Hosp. 2017 Nov 16;34(5):1482-1488. doi: 10.20960/nh.1220*.)*

Several patent applications and granted patents have described use of ablation in the stomach for treatment of various medical disorders, including obesity.

US patent #6,427,089 by Knowlton teaches treating obesity by use of an endoluminal device incorporating a microwave antenna for inducing contraction of gastric volume or reducing the distensibility of the stomach.

US patent #7,282,050 by Starkebaum et al. teaches ablation of the exterior of the stomach to alter gastric function for the treatment of obesity.

US patent #7,468,060 by Utley et al. teaches tightening of the pyloric sphincter using ablation and/or mediating receptive relaxation of muscles in the stomach, for treating obesity and other disorders.

US patent #8,641,711 by Kelly et al. teaches treatment of metabolic syndrome or obesity by controlled ablation of tissue within the wall of the gastro-intestinal tract, mainly in the jejunum.

US application 2006/0217698 A1 by Starkebaum el al. teaches treating obesity by endoluminal ablation of gastric mucosa for reduction of ghrelin production, or ablation of the submucosa, nerves, or muscle tissue to induce gastroparesis.

US application 2005/0240239 A1 by Boveja el al. teaches treating obesity by endoluminal ablation of the intrinsic gastric pacemaker area and optional implantation of an artificial pacemaker for slower pacing of the stomach.

All the above suggest creation of focal or area lesions, and do not look at the stomach as a whole. Such an approach may cause damage to the gastric wall by creating large lesions. A more physiologically oriented approach is needed.

Since, as described above, gastric motility is mediated by the interstitial cells of Cajal, obesity may be viewed as a tachy-arrhythmia of the stomach, and as such may be treated by controlling propagation of signals through the stomach wall using thin ablation lines, which may alter the behavior of the stomach as a whole.

Such an alternative approach was described in granted US patents #9,883,906 and #9,179,963 by Ben-Ezra et al.

Additional, more specific embodiments, including detailed attributes of devices for treating gastro-intestinal diseases according to the above approach are described herein.

The same devices may be used for inducing contraction of tissue along the ablated lines, to modify the mechanical behavior of the stomach, such as its distensibility, motility, and capability to propagate gastric contents.

Other gastrointestinal related disorders, such as constipation, gastroparesis, gastroesophageal reflux disease, dumping syndrome, bloating, belching, nausea, cyclic vomiting, binge eating disorder, peptic ulcer disease, gastritis, anorexia nervosa, bulimia, irritable bowel syndrome, diabetes, and more, may also be treated using similar approaches, as detailed below.

Of note, devices described herein with regard to the stomach, are meant to generally apply to other gastrointestinal organs as well.

US 5 125 928 A relates to a RF ablation catheter for removing athero-stenotic lesions or modifying the tissue characteristics of the interior walls of selected blood vessels is described. The catheter is characterized in having a tip member which during the initial placement of the catheter within the vascular system so that the distal tip member is disposed in a working relation with the lesion to be removed, means are provided for increasing the cross-sectional profile of the tip members to thereby cause it to move into engagement with the lesion to be excised. The application of an RF voltage across a pair of bipolar electrodes is used to create an electric arc for effecting the cutting action, or alternatively, just sufficient RF energy to sear or otherwise alter the tissue surfaces engaged by the distal tip member.

US 2006/0293643 A1 relates to a surgical device integrating a suction mechanism with a coagulation mechanism is provided for improving lesion creation capabilities. The device comprises an elongate member having an insulative covering attached about means for coagulating soft tissue. Openings through the covering expose regions of the coagulation-causing elements and are coupled to lumens in the elongate member which are routed to a vacuum source. A fluid source to passively transport fluid along the contacted soft tissue surface may be provided in order to push the maximum temperature deeper into tissue.

US 2004/186467 A1 relates to an apparatus and methods for maintaining contact between tissue and diagnostic and therapeutic elements.

### SUMMARY OF THE INVENTION

The current invention relates to any situation in which it may be beneficial to deliver bipolar energy to a target material or tissue, using a flexible, or deformable, member. This may typically be used in medical applications such as various tissue ablation procedures, including but not limited to cardiac ablation performed for rhythm control, endoscopic surgery on the **GI** tract for hemorrhage control or polyp removal, ablation in the stomach for treating obesity or gastric motility disorders e.g. dumping syndrome, or anywhere in the **GI** tract for various forms of irritative bowel syndrome, constipation, etc., bladder ablation for overactive bladder or other micturition disorders, endometrial ablation for menorrhagia etc. It may also be useful in many other medical or non-medical applications, such as, but not limited to, welding of polymers (these would typically be polymers containing polar molecules, e.g. PVC or polyurethane) for example during fusion of pipes, sheets, or balloons for various industrial purposes. Specific embodiments described herein are related to the field of gastroenterology, and more specifically to the modulation of the activity of gastro-intestinal organs using minimally invasive, endoscopic means, to alleviate obesity, constipation, or other gastro-intestinal related conditions.

The invention is defined in independent claim 1, with optional features set out in the dependent claims.

### BRIEF DESCRIPTION OF THE DRAWINGS

**FIG. 1A** - is a simplified schematic top view of a flexible bipolar electrode according to an embodiment of the current invention.
**FIG. 1B** - schematic three-dimensional depiction of a flexible bipolar electrode according to an embodiment of the current invention.
**FIG. 2A** - is a simplified schematic side view of a flexible spiral bipolar electrode according to an embodiment of the current invention.
**FIG. 2B** - is a simplified schematic top view of a flexible spiral bipolar electrode according to an embodiment of the current invention.
**FIG. 2C** - is a simplified schematic top view of a flexible spiral bipolar electrode and contact points according to an embodiment of the current invention.
**FIG. 2D** - is a simplified schematic cross section (at line D in figs. 2A-2C) of a flexible spiral bipolar electrode according to an embodiment of the current invention.
**FIG. 2E** - is a simplified schematic cross section (at line E in figs. 2A-2C) of a flexible spiral bipolar electrode according to an embodiment of the current invention.
**FIG. 3A** **-** is a simplified schematic cross section (at line D in figs. 2A-2C) of a flexible spiral bipolar electrode according to an embodiment of the current invention.
**FIG. 3B** - is a simplified schematic cross section (at line E in figs. 2A-2C) of a flexible spiral bipolar electrode according to an embodiment of the current invention.
**FIG. 4A** **-** is a simplified schematic cross section (at line D in figs. 2A-2C) of a flexible spiral bipolar electrode according to an embodiment of the current invention.
**FIG. 4B** - is a simplified schematic cross section (at line E in figs. 2A-2C) of a flexible spiral bipolar electrode according to an embodiment of the current invention.
**FIG. 4C** - is a simplified schematic three-dimensional depiction of a flexible spiral bipolar electrode according to an embodiment of the current invention.
**FIG. 5** **-** is a simplified schematic three-dimensional depiction of a flexible spiral bipolar electrode according to an embodiment of the current invention.
**FIG. 6A** **-** is a simplified schematic cross section of a flexible spiral bipolar electrode according to an embodiment of the current invention.
**FIG. 6B** **- is** a simplified schematic three-dimensional depiction of a flexible spiral bipolar electrode according to an embodiment of the current invention.
**FIG. 7A** - is a simplified schematic cross section of a manufacturing jig according to an embodiment of the current invention.
**FIG. 7B** - is a simplified schematic top view of a manufacturing jig according to an embodiment of the current invention.
**FIG. 8A** - is a simplified schematic anterior view of a human stomach.
**FIG. 8B** - is a simplified schematic cross section through the wall of a human stomach.
**FIG. 9A** - is a simplified schematic anterior view of a human stomach with an ablation pattern according to an embodiment of the current invention.
**FIG. 9B** - is a simplified schematic anterior view of a human stomach with an ablation pattern according to an embodiment of the current invention.
**FIG. 9C** - is a simplified schematic anterior view of a human stomach with an ablation pattern according to an embodiment of the current invention.
**FIG. 9D** - is a simplified schematic anterior view of a human stomach with an ablation pattern according to an embodiment of the current invention.
**FIG. 9E** - is a simplified schematic anterior view of a human stomach with an ablation pattern according to an embodiment of the current invention.
**FIG. 9F** - is a simplified schematic anterior view of a human stomach with an ablation pattern according to an embodiment of the current invention.
**FIG. 9G** - is a simplified schematic anterior view of a human stomach with an ablation pattern according to an embodiment of the current invention.
**FIG. 9H** - is a simplified schematic anterior view of a human stomach with an ablation pattern according to an embodiment of the current invention.
**FIG. 10A** - is a simplified schematic anterior view of a human stomach with an ablation pattern according to an embodiment of the current invention.
**FIG. 10B** - is a simplified schematic anterior view of a human stomach with an ablation pattern according to an embodiment of the current invention.
**FIG. 10C** - is a simplified schematic anterior view of a human stomach with an ablation pattern according to an embodiment of the current invention.
**FIG. 10D** - is a simplified schematic anterior view of a human stomach with an ablation pattern according to an embodiment of the current invention.
**FIG. 11A** - is a simplified schematic anterior view of a human stomach with an ablation pattern according to an embodiment of the current invention.
**FIG. 11B** - is a simplified schematic anterior view of a human stomach with an ablation pattern according to an embodiment of the current invention.
**FIG. 11C** - is a simplified schematic anterior view of a human stomach with an ablation pattern according to an embodiment of the current invention.
**FIG. 11D** - is a simplified schematic anterior view of a human stomach with an ablation pattern according to an embodiment of the current invention.
**FIG. 11E** - is a simplified schematic anterior view of a human stomach with an ablation pattern according to an embodiment of the current invention.
**FIG. 12A** - is a simplified schematic frontal view of a global pattern ablation device according to **an** embodiment of the current invention.
**FIG. 12B** - is a simplified schematic frontal view of a global pattern ablation device according to **an** embodiment of the current invention.
**FIG. 12C** - is a simplified schematic frontal view of a global pattern ablation device according to an embodiment of the current invention.
**FIG. 13** - is a simplified schematic frontal view of a regional pattern ablation device according to an embodiment of the current invention.
**FIG. 14A** - is a simplified schematic frontal view of a local pattern ablation device according to an embodiment of the current invention.
**FIG. 14B** **-** is a simplified schematic frontal view of a local pattern ablation device according to an embodiment of the current invention.
**FIG. 15** - is a simplified schematic anterior view a human large intestine.
**FIG. 16A** - is a simplified schematic anterior view of an ablation pattern in a human large intestine.
**FIG. 16B** - is a simplified schematic anterior view of an ablation pattern in a human large intestine.
**FIG. 17** - is a simplified schematic anterior view of an ablation device in a human large intestine.
**FIG. 18A** - is a simplified three-dimensional view of an ablation device with spiral bipolar and parallel electrodes according to an embodiment of the current invention.
**FIG. 18B** - is a simplified schematic side view of an ablation device with spiral bipolar and parallel electrodes in its deployed state according to an embodiment of the current invention.
**FIG. 18C** - is a simplified schematic side view of an ablation device with spiral bipolar and parallel electrodes in its crimped state according to an embodiment of the current invention.

### DETAILED DESCRIPTION OF THE INVENTION

### FLEXIBLE BIPOLAR ELECTRODE

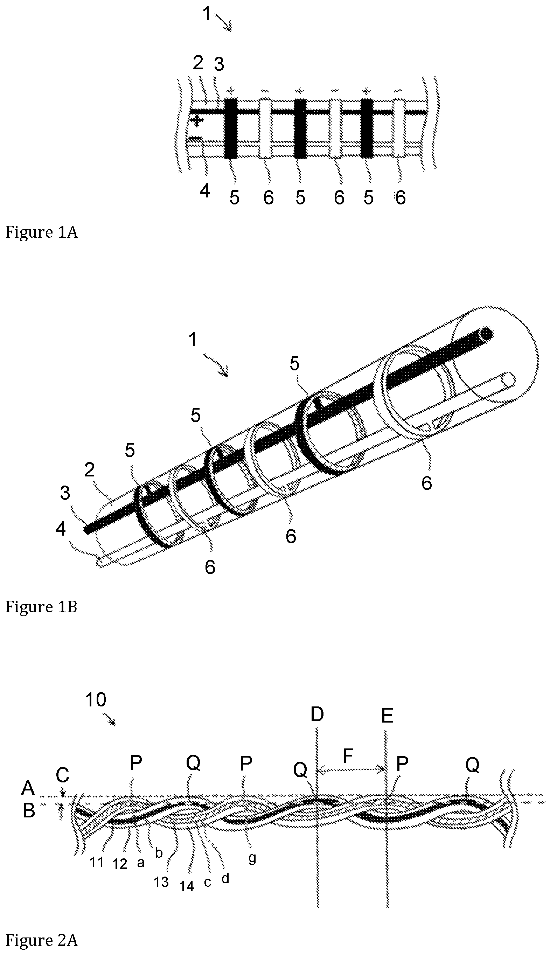

FIG. 1A is a schematic top view of an embodiment of the current invention. More particularly, shown in FIG. 1A is elongate flexible bipolar electrode 1, which may comprise a non-conductive, optionally flexible "core" 2, and at least two conductors 3 and 4, wherein each may comprise multiple, alternating contacts 5 and 6.

As seen in FIG. 1A, connecting each of conductors 3 and 4 to an opposite electrical pole may result in contacts 5 and 6 representing alternating positive and negative poles along electrode 1. Thus, electricity flowing through the tissue or target material between each adjacent pair of opposite poles 5 and 6 may warm the tissue and result with an effect, which may depend on the power delivered, as well as on the tissue and electrode properties.

FIG. 1B is a schematic three-dimensional sketch of a possible embodiment of the concept described in FIG. 1A.

More particularly, shown in FIG. 1B is elongate flexible bipolar electrode 1, which may comprise a non-conductive, optionally flexible "core" 2, and at least two conductors 3 and 4, wherein each may comprise multiple, alternating contacts 5 and 6.

Core 2 may be made of a flexible non-conductive material, preferably heat resistant, for example it may be made of silicone, polyurethane, pvc, polyimide, polyester, etc.

Conductors 3 and 4 and contacts 5 and 6 may be made of a conductive material such as copper, silver, gold, stainless steel, nickel, NiTi, etc.

The embodiment shown in FIGS. 1A and 1B may be manufactured by injection molding core 2 over conductors 3 and 4. Alternatively it may be manufactured by dipping conductors 3 and 4 in the material of core 2, to be followed by exposing the contacts 5 and 6. In some embodiments, manufacturing may be by 3D printing or co-extrusion of core 2 simultaneously with conductors 3 and 4.

A preferred embodiment of the current invention is shown in FIGS. 2A-2E.

As will be described in detail below, the invention comprises an elongate flexible spiral bipolar electrode 10 made of two insulated conductors, twisted together to form a twisted pair, a braid, or other similar structure with the insulation removed at one or more locations where the conductors are exposed, forming a bipolar pair on at least one of the sides of the electrode, such that the exposed areas of the two conductors form a line of alternating electrical poles.

FIG. 2A is a schematic side view of a preferred embodiment of the electrode.

More particularly, shown in FIG. 2A is elongate spiral bipolar electrode 10 which may comprise conductor 11 which may have a diameter "a" and may be covered by insulation layer 12 which may have a thickness "b", and conductor 13 which may have a diameter "c" and may be covered by insulation layer 14 which may have a thickness "d". For clarity, insulation layers 12 and 14 are depicted transparent, so conductors 11 and 13 are seen through them. Conductor 11 together with insulation layer 12 and conductor 13 together with insulation layer 14 may sometimes herein be collectively referred to as "wires" 11 and 13.

Wires 11 and 13 may be wound around each other, preferably at the same direction (either clockwise or counterclockwise), such that they may form a twisted pair, a braid, or other similar embodiment along which they may be symmetrically dispersed. Twisting or braiding maintains close proximity of the wires down the length. This mechanical augmentation can be achieved through adhesive bonding of the two wires, fusion of the two insulation materials, etc.

Typically, diameters "a" and "c" of conductors 11 and 13 may be equal, and thicknesses "b" and "d" of insulation layers 12 and 14 may be equal. However, in some embodiments, these parameters may be intentionally not equal to each other.

Gap "g" marks the distance between the surfaces of insulation layers 12 and 14. Although depicted here as a significant distance, gap g may be very small, or zero, i.e. no gap may exist between insulation layers 12 and 14. In other words, insulation layer 12 and 14 may touch each other. Furthermore, in some embodiments the insulation materials may be adhesively, chemically, thermally, or otherwise bonded or fused together, or originally formed as a single element.

Although not depicted in FIG. 2A, in some embodiments, additional conductors or spiral elements may be incorporated into electrode 10, for modifying its mechanical, electrical, or other properties. For example, electrode 10 may comprise a total of three, four, or more wires instead of just two. All or just some of these wires may be electrically active. Alternatively, the additional wires may be used only for ensuring a specific dimensional gap between the electrically active wires 11 and 13, or they may serve to affect the strength, durability, or other properties of electrode 10.

Dashed line A, parallel to the general longitudinal direction of electrode 10, marks the outermost "peaks" of the spiraled wires before removal of insulation.

Dashed line B, parallel to line A, may be at a distance C from it, and marks the extent to which insulation layers 12 and 14 may be removed according to the current disclosure. Thus, in the area seen in FIG. 2A between lines A and B, insulation layers 12 and 14 may be removed, leaving conductors 11 and 13 exposed, such that each of conductors 11 and 13 may be in contact with the target tissue or material at tissue contact points Q and P respectively.

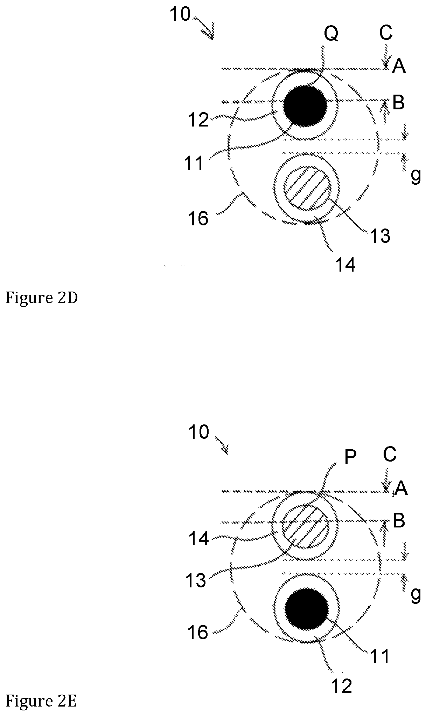

Line D, generally perpendicular to line A, passes through a "peak" and tissue contact point Q of conductor 11, and marks the location of the cross section shown in FIG. 2D.

Line E, generally perpendicular to line A, and parallel to line D at a distance of F from it, passes through a "peak" and tissue contact point P of conductor 13, and marks the location of the cross section shown in FIG. 2E.

Distance F between lines D and E may be the distance between two intermittent poles of electrode 10. Distance F may be a function of the frequency, or pitch, of electrode 10, and may typically be equal to half the inverse of this pitch. For example, in a case where the pitch of the spiral of each of conductors 11 and 13 may be 8 coils per inch, distance F may be 1/16 of an inch, or 1.5875mm.

Specifications of the wires, diameters, insulation type and thickness, pitch, hardness, etc. may vary according to the application. Typical wires used for electrode 10 may be similar to magnet wire.

For example, typical specifications for wires used in an electrode 10 for a medical device application, such as a device intended for ablation in a urinary bladder, may be similar to the following:

Conductor material: may typically be fully annealed, electrolytically refined copper, or aluminum, or any other appropriate material.

Conductor diameter: typically, 0.001" - 1" or greater, preferably 0.005" - 0.020"

Insulation material: may typically be polyvinyl formal (Formvar), polyurethane, polyamide, polyester, polyester-polyimide, polyamide-polyimide (or amide-imide), polyimide, silicone, or any other appropriate material.

Insulation thickness: typically, 0.0001" - 0.005" or greater, preferably 0.0005" - 0.001".

Whole electrode diameter: typically, 0.001" - 1" or greater, preferably 0.002" - 0.050"

Pitch: typically, 1 - 500 coils per inch or greater, preferably 5 - 20 coils per inch.

The dimensions of gap g may typically be 0-0.1", preferably 0-0.01".

Furthermore, adhesion or fusion of the insulation materials can be used to augment the retention force of the wires twisted together.

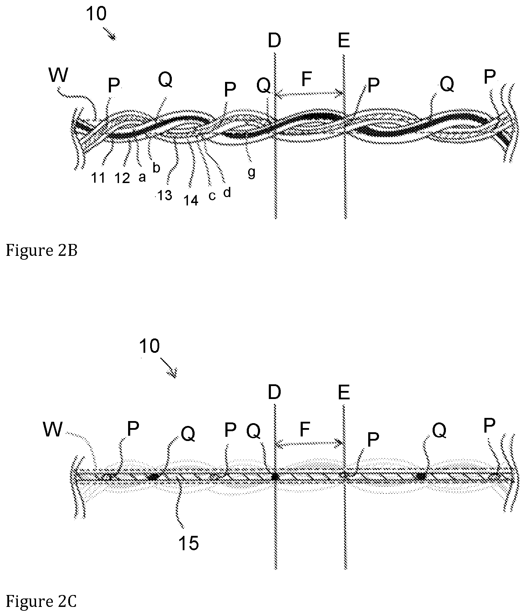

FIG. 2B is a schematic top view of the preferred embodiment of the electrode described above and in FIG. 2A.

More particularly, shown in FIG. 2B is elongate spiral bipolar electrode 10 which may comprise conductor 11 which may have a diameter a and may be covered by insulation layer 12 which may have a thickness b, and conductor 13 which may have a diameter c and may be covered by insulation layer 14 which may have a thickness d. In the interest of clarity, insulation layers 12 and 14 are depicted transparent, so conductors 11 and 13 are seen through them.

Rectangular dashed box "W", having a longitudinal axis parallel to the general longitudinal direction of electrode 10, marks the outermost area (as seen from this top view) of electrode 10 where insulation layers 12 and 14 may be removed, leaving conductors 11 and 13 exposed, such that each of conductors 11 and 13 may be in contact with the target tissue or material at tissue contact points Q and P respectively.

Line D, generally perpendicular to longitudinal axis of rectangular box "W", passes through a "peak" and tissue contact point Q of conductor 11, and marks the location of the cross section shown in FIG. 2D.

Line E, generally perpendicular to longitudinal axis of rectangular box "W", and parallel to line D at a distance of F from it, passes through a "peak" and tissue contact point P of conductor 13, and marks the location of the cross section shown in FIG. 2E.

FIG. 2C is a schematic top view of the preferred embodiment of the electrode described above and in FIGS. 2A and 2B, also showing tissue contact points P and Q.

More particularly, FIG. 2C shows elongate spiral bipolar electrode 10 including all elements shown in FIG. 2B. In addition, FIG. 2C includes rectangular dashed box W, having a longitudinal axis parallel to the general longitudinal direction of electrode 10, which marks the outermost area of electrode 10 where insulation layers 12 and 14 may be removed, as seen from this top view, leaving conductors 11 and 13 exposed, such that each of conductors 11 and 13 are in contact with the target tissue or material at tissue points P and Q.

Tissue contact points P and Q are shown forming a line of intermittent alternating poles. Elongate ablation area 15 marks the area of tissue effect or ablation, which may result from delivery of energy, e.g., AC, DC, RF, microwave, etc. to conductors 11 and 13 when in contact with a target tissue. The current may flow through the tissue, between each adjacent tissue contact point pairs P and Q, thus creating ablation lesions that coalesce to form elongate ablation area 15, which may typically be substantially continuous.

Line D, generally perpendicular to the longitudinal axis of rectangular box W, passes through a "peak" and tissue contact point Q of conductor 11, and marks the location of the cross section shown in FIG. 2D.

Line E, generally perpendicular to longitudinal axis of rectangular box W, and parallel to line D at a distance of F from it, passes through a "peak" and tissue contact point P of conductor 13, and marks the location of the cross section shown in FIG. 2E.

FIG. 2D is a schematic cross section of the preferred embodiment of the electrode described above and in FIGS. 2A-2C.

More particularly, FIG. 2D is a cross section through line D of FIGS. 2A-2C. Seen in FIG. 2D is elongate spiral bipolar electrode 10 comprising conductors 11 and 13. Conductor 11, covered by insulation layer 12, may be located at the upper part of electrode 10 in this cross section. Conductor 13, covered by insulation layer 14, may be at the bottom part of electrode 10 in this cross section. Perimeter 16 marks the outer margins of the circle formed by the spiraled wires 11 and 13.

Also seen in this figure is gap "g", which as mentioned above, may be very small, or even equal to zero.

Dashed line A marks the outermost "peaks" of the spiraled insulated wires before removal of insulation.

Line B, parallel to line A, may be at a distance C from it, and marks the extent to which insulation layers 12 and 14 may be removed in this embodiment. Thus, in the area seen in FIG. 2D between lines A and B, insulation layer 12 may be removed, leaving conductor 11 exposed, such that it may be in contact with the target tissue or material at tissue point or points Q.

FIG. 2E is a schematic cross section of the preferred embodiment of the electrode described above and in FIGS. 2A-2D.

More particularly, FIG. 2E is a cross section through line E of FIGS. 2A-2C. Seen in FIG. 2E is elongate spiral bipolar electrode 10 comprising conductors 11 and 13. Conductor 13, covered by insulation layer 14, may be located at the upper part of electrode 10 in this cross section. Conductor 11, covered by insulation layer 12, may be at the bottom part of electrode 10 in this cross section.

Perimeter 16 marks the outer margins of the circle formed by the spiraled wires 11 and 13. Also seen is Gap "g" between the surface of insulation layers 12 and 14.

Dashed line A marks the outermost "peaks" of the spiraled wires before removal of insulation.

Line B, parallel to line A, may be at a distance C from it, and marks the extent to which insulation layers 14 may be removed according to the current disclosure. Thus, in the area seen in FIG. 2E between lines A and B, insulation layer 14 may be removed, leaving conductor 13 exposed, such that it may be in contact with the target tissue or material at tissue point or points P.

In the current embodiment, the exposed side of the wires should be oriented towards the tissue, so that contact points P and Q may be in contact with the tissue, forming a line of alternating poles, so that when an electrical current is applied to conductors 11 and 13, the tissue may be ablated.

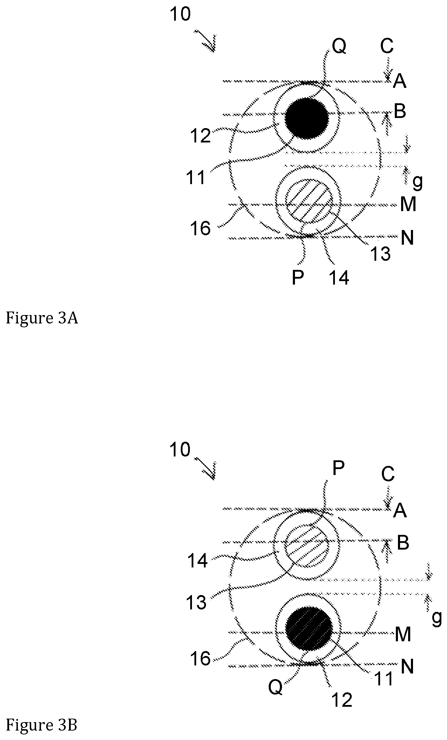

In some embodiments, exposure of conductors 11 and 13 may be performed on two or more sides of electrode 10. This is shown in FIGS. 3A and 3B which are identical to FIGS. 2D and 2E respectively in all details, apart from also showing dashed lines "M" and "N" which denote another area of removal of insulation and exposure of conductors. This increases the chances that the conductors will be in contact with tissue even if the electrode is not oriented as intended.

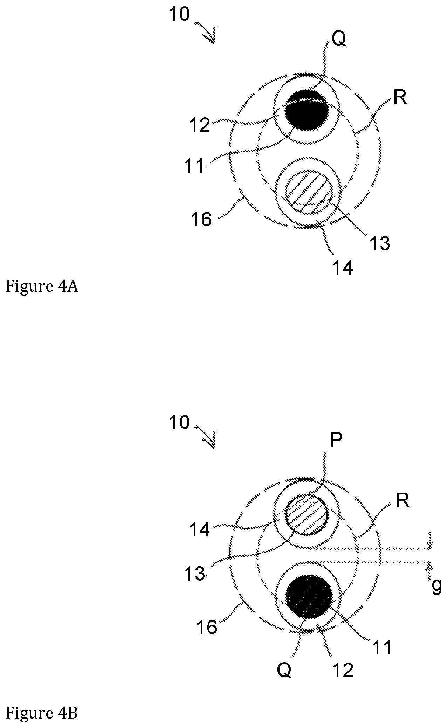

Exposure of conductors 11 and 13 may optionally be performed for example on three, four, or more sides of electrode 10. In fact, insulation may be removed from all around electrode 10, and conductors 11 and 13 may still be insulated from each other, as sufficient insulation may remain between them. This is depicted in FIGS. 4A-4C.

FIG. 4A is a schematic cross section of an embodiment of electrode 10. More particularly, seen in FIG. 4A is elongate spiral bipolar electrode 10 comprising conductors 11 and 13. Conductor 13, covered by insulation layer 14, may be located at the upper part of electrode 10 in this cross section. Conductor 11, covered by insulation layer 12, may be at the bottom part of electrode 10 in this cross section.

Perimeter 16 marks the outer margins of the circle formed by the spiraled wires 11 and 13.

Circular dashed line R denotes an area of removal of insulation and exposure of conductors, which in the embodiment shown may be all around electrode 10.

The parts of insulation layers 12 and 14 outside dashed line "R" may be removed, while the parts of insulation layers 12 and 14 enclosed within dashed line "R", may not be removed, and may remain in the electrode, separating conductors 11 and 13, even when electrode 10 is bent or twisted. This may be extremely beneficial, as the exact orientation of electrode 10 relative to the tissue no longer matters, as long as there is contact between electrode 10 and the tissue. At any orientation, the parts of exposed conductors 11 and 13 in contact with the tissue may form a line of alternating poles and enable bipolar energy delivery to the tissue along that line.

FIG. 4B is a schematic cross section of the same embodiment of electrode 10, at an adjacent location along electrode 10 to that shown in FIG. 4A. FIG. 4B is essentially the same as

FIG. 4A with the difference being that conductor 11 is seen here at the bottom and conductor 13 at the top.

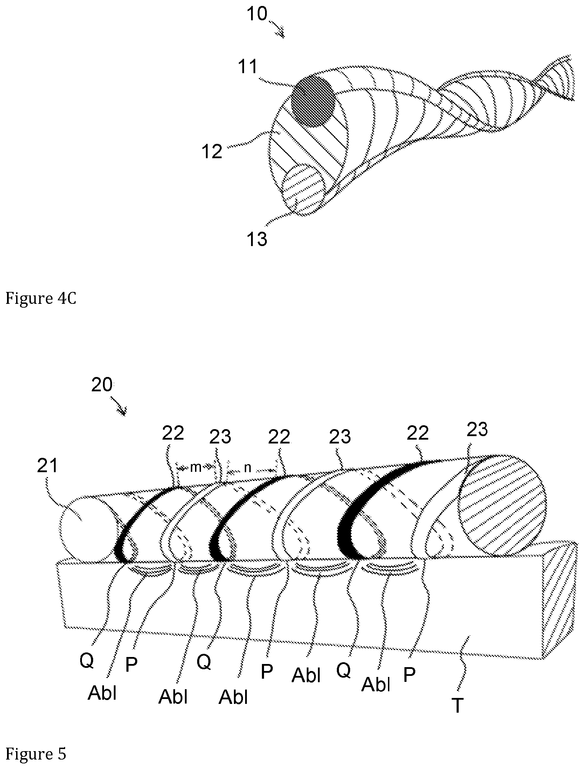

FIG. 4C is a three-dimensional depiction of the same embodiment of electrode 10. In this illustration, insulation layers 12 and 14 are shown as fused to each other and are marked by the number 12 only.

In all of the embodiments described herein, conductors 11 and 13 may each be made of a single strand or filar, or of several strands or filars braided or twisted together. A braided or twisted conductor may be more flexible and durable than a single strand conductor but removing the insulation from it may be more difficult.

FIG. 5 is a schematic three-dimensional sketch of yet another possible embodiment of the concept described in FIGS. 4A-4C.

More particularly, shown in FIG. 4 is elongate flexible spiral bipolar electrode 20, which is shown in contact with a piece of target tissue "T". In the interest of clarity, electrode 20 is depicted semi-transparent and a longitudinal section of tissue T is shown along its line of contact with electrode 20. Electrode 20 may comprise a non-conductive, optionally flexible core 21, and at least two spiral conductors 22 and 23, at a distance "m" from each other. The distance between two adjacent coils may be "n", which may typically be equal to "m", but may optionally be different. As with the embodiment shown in FIGS. 4A-4C, spiral conductors 22 and 23 may be exposed along their whole length, but since they only come into contact with the target tissue or material along one side of core 21, tissue contact points "P" and "Q" may form a linearly alternating series of poles. Ablated areas "Abl" between tissue contact points Q and P denote areas that may be ablated or otherwise affected by delivering energy to electrode 20 and may typically coalesce to form a substantially continuous line of tissue effect along the course of electrode 20.

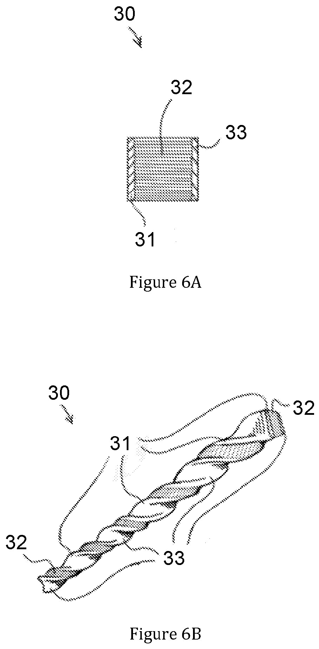

Yet another embodiment, is electrode 30 which may be manufacturable using 3D printing or extrusion, followed by twisting, is shown in FIGS. 6A and 6B.

In this embodiment, an insulator core is coated by conductors on two of its sides. The structure is then twisted along its longitudinal axis to achieve alternating poles along electrode 30.

More particularly, FIG. 6A is a cross section of electrode 30 showing from left to right: conductor 31, insulator core 32, conductor 33.

FIG. 6B is a schematic three-dimensional depiction of electrode 30.

More particularly, FIG. 6B shows electrode 30 comprising insulator core 32 with conductors 31 and 33 on both sides, twisted to form alternating poles.

In some embodiments, the current invention may be used to deliver energy to whole areas instead of in a linear fashion. This may for example be achieved by placing any of the above described embodiments side by side, optionally in a staggered positioning, so as to create a surface of alternating poles (in a "chess board" pattern).

In use, any of the above described embodiments may be brought into contact with a target tissue or material. This may be done in any way as known in the art, including but not limited to direct topical application to skin or any other external tissue, endoscopically, percutaneously, surgically, etc. and with the aid of catheters and/or balloons as necessary.

Various forms of electrical energy may be delivered to the electrode. For example, typical power settings for ablating lines in a human urinary bladder using any of the above embodiments may be Radio Frequency energy at approximately 500KHz, 30-50 Watts for 1-3 seconds.

### Manufacturing methods

A manufacturing method, which is not part of the invention specifically relevant to the embodiment described in FIGS. 5 and 6 may be three-dimensional printing of conductive ink over an elongate polymer, which may serve as core 21.

The advantage of using this method is that post processing for removal of insulation is not required. On the other hand, it may be time consuming, expensive, and it may also be difficult to achieve accurate and dependable electrical properties using this method.

Going back to the embodiments described in FIGS. 2A-4C, various manufacturing methods may be available.

Methods for manufacturing the wires of electrode 10 are well known in the art, as materials and methods similar to those used for manufacturing magnet wire may be used for insulating and twisting wires 11 and 13. Mechanical retention of the two wires can be augmented through adhesive bonding of the two wires, fusing of the two insulation materials, etc.

After twisting together wires 11 and 13, insulation layers 12 and 14 may be removed using any of the following methods:
Mechanical abrasion:
This may include but is not limited to grinding, sanding, bead-blasting, or filing using solid abrasives.

Alternatively, insulation may be removed using sharp blades. Yet alternatively, jets of fluid may be used to abrasively remove insulation.

### Thermal abrasion:

Electrical or laser energy may be used to heat and remove insulation.

### Chemical abrasion:

Abrasive chemicals including acids may be used to erode the insulation and remove it.

As described previously, removal of insulation using any of the above methods may be performed on one side, two or more sides, or all sides of electrode 10.

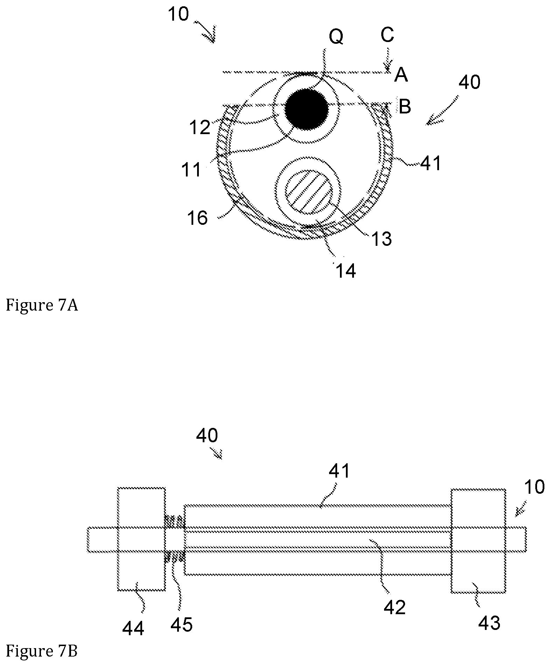

To enable accurate and easy removal of insulation, various methods and tools may be employed. By way of example, a jig that may be used for this purpose is described in FIGS. 7A and 7B.

FIG. 7A is a schematic simplified cross section of jig 40 used for manufacturing of electrode 10.

More particularly, FIG. 7A includes the exact same details as FIG. 2D, and in addition it also shows jig 40, which may have a C-shaped cross section. Shaft 41 of jig 40 is seen surrounding electrode 10 from all sides apart from its upper side. The open edges of jig 40 may reach dashed line B. The area of electrode 10 between dashed lines A and B may not be covered by jig 40. This may enable accurate exposure of the uncovered area of electrode 10 to be exposed to any of the abrasion methods mentioned above, for removal of insulation layers 12 and 14, only in this area.

FIG. 7B is a schematic simplified top view of jig 40 used for manufacturing of electrode 10.

More particularly, FIG. 7B shows, from right to left: electrode 10, distal attachment 43, jig aperture 42, jig shaft 41, tension means 45, and proximal attachment 44.

Jig aperture 42 is the open part of the C-shaped cross section of jig shaft 41. Its ends are marked with sashed lines, as it may reach both ends of jig shaft 41. Distal attachments 43 and proximal attachment 44 may be any type of fixture that may enable cinching electrode 10, and adjustment of the degree of force and tension applied to electrode 10, such as a Tuohy Borst valve. Tension means 45 may be a spring or any other mean of applying tension to electrode 10, preferably in an adjustable manner.

During manufacturing of electrode 10, jig 40 may be used to remove insulation at a desired area using the following method: electrode 10 (prior to removal of insulation) may be threaded into shaft 41 of jig 40, and cinched at both ends by attachments 43 and 44. The degree of tension may be adjusted using attachments 43 and 44 and tension means 45, so that electrode 10 is taut but not excessively stretched. This may for example be achieved by measuring the tension using force gauge, or by tension means 45 being pre-set to a specific force. Once properly mounted in the jig, abrasion processes may be applied to the exposed are of electrode 10, accessible through jig aperture 42.

Of note, jig shaft 41 may be made of a rigid and durable material such as stainless steel to prevent it from eroding during use. It may alternatively or additionally be comprised of materials that may provide protection to the rest of electrode 10 from thermal or chemical effects such as various types of polymers including but not limited to polycarbonate, PVC, etc.

In some embodiments, jig shaft 41 may comprise two or more apertures to enable removal of insulation from multiple sides of electrode 10.

In some embodiments, electrode 10 may be rotated inside jig shaft 41 during the insulation removal process, so that multiple areas of its circumference may be exposed to the process, and insulation may be removed from all or part of its circumference.

In some embodiments, removal of insulation may be done intermittently, i.e. only in various areas along electrode 10. A jig 40 having several intermittent jig apertures 41 may be used for this purpose.

Alternatively, as shown in FIGS. 6A and 6B, manufacturing of electrode 30, which may be functionally identical to electrodes 10 and 20, may be achieved by a specific method in which bare conductors are twisted along a non-conductive core. This method may be used to produce an electrode similar to the one shown in FIGS. 4A-4C, in a quicker, cheaper, and more efficient way than removing insulation from twisted wires or 3D printing over a non-conductive core. Such process may be done as a co-extrusion of both core 32 and conductors 31 and 33, or each of core 32 and conductors 31 and 33 may be extruded separately, and then they may be braided together to achieve a structure similar to that described in FIGS. 3B and 4.

In some embodiments, the electrodes described herein may be used for sensing electrical signals and tissue impedance.

### TREATING MEDICAL CONDITIONS INCLUDING OBESITY AND CONSTIPATION

The current invention further includes devices for treating obesity and other gastrointestinal conditions, by producing certain ablation patterns on the gastric wall. The ablation patterns may comprise ablation lesions through which propagation of electrical, neural, or hormonal activity may be reduced or otherwise modified. By modifying this propagation, the ablation patterns are intended to modify the behavior of the organ as a whole. Areas of the stomach or organ, which may be completely or partially isolated from each other by the ablation patterns, will herein be referred to as "partitions".

### Global ablation patterns

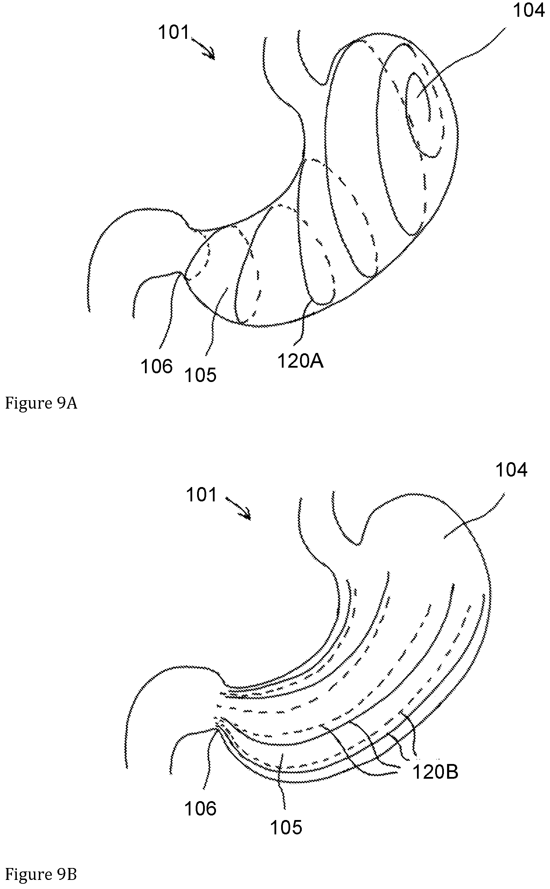

In some embodiments, depicted in FIGS. 9A-9F, the ablation patterns may be global. The meaning of "global" here is that the pattern may be dispersed over substantially the whole organ.

Of note, the patterns may typically comprise thin lines, relative to the gaps between the lines, such that although the lesions extend over the whole organ, most of the organ's surface may remain free of lesions, i.e. unablated.

Examples of such ablation patterns are shown in FIGS. 9A-9F in a stomach, although the treatment may be applied to many other organs. As will be explained below, the "global" patterns may extend over substantially all the organ, or at least over a large part of it.

In some embodiments, at least three fourths of the stomach area may be treated (i.e. no partition may be larger than a fourth of the total inner surface area of the stomach). In other embodiments, at least two thirds of the stomach area may be treated (i.e. no partition may be larger than a third of the total inner surface area of the stomach). In yet other embodiments, at least a half of the stomach area may be treated (i.e. no partition may be larger than a half of the total inner surface area of the stomach).

More particularly, FIG. 9A is a schematic anterior view of a stomach 101, with an ablation pattern 20A ablated on its wall. Pattern 120A is a substantially continuous spiral line extending from fundus 104 to antrum 105 or pylorus 106. In some embodiments, pattern 120A may comprise several such lines, parallel to each other.

Such a pattern may slow both axial and circumferential propagation through the gastric wall, which may reduce the rate of gastric emptying.

FIG. 9B is a schematic anterior view of a stomach 101, with an ablation pattern 120B ablated on its wall. Pattern 120B may comprise several substantially continuous lines, parallel to the longitudinal axis of the stomach, extending from fundus 104 to antrum 105 or pylorus 106.

Such a pattern may slow circumferential propagation through the gastric wall, which may reduce the efficacy of peristaltic contractions, and prolong gastric emptying time.

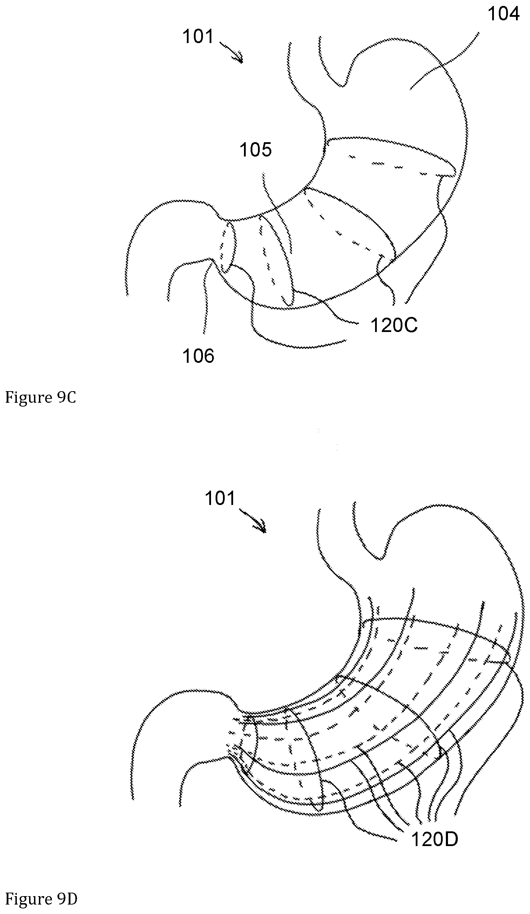

FIG. 9C is a schematic anterior view of a stomach 101, with an ablation pattern 120C ablated on its wall. Pattern 120C may comprise several substantially continuous lines, perpendicular to the longitudinal axis of the stomach, and optionally spanning the whole circumference of the stomach, which may be distributed from the area of fundus 104 to antrum 105 or pylorus 106.

Such a pattern may slow axial propagation through the gastric wall.

FIG. 9D is a schematic anterior view of a stomach 101, with an ablation pattern 120D ablated on its wall. Pattern 120D is essentially a combination of patterns 120B and 120C, thus creating a grid of longitudinal and perpendicular lines.

Such a pattern may slow both axial and circumferential propagation through the gastric wall.

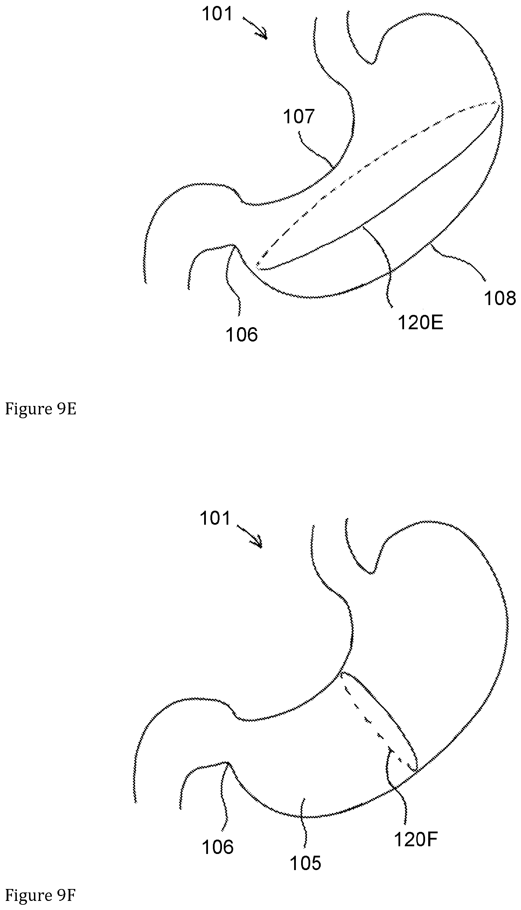

FIG. 9E is a schematic anterior view of a stomach 101, with an ablation pattern 120E ablated on its wall. Pattern 120E may comprise a substantially continuous line generally parallel to the longitudinal axis of the stomach, and optionally spanning the whole circumference of the stomach, essentially separating greater curve 108 from the lesser curve 107 and pylorus 106.

In some embodiments, pattern 120E may comprise several such lines, parallel to each other.

Such a pattern may slow propagation through the gastric wall, of signals from greater curve 108 to pylorus 106.

FIG. 9F is a schematic anterior view of a stomach 101, with an ablation pattern 120F ablated on its wall. Pattern 120F may comprise a substantially continuous line generally perpendicular to the longitudinal axis of the stomach, and optionally spanning the whole circumference of the stomach, essentially separating greater curve 108 from the lesser curve 107 and pylorus 106.

In some embodiments, pattern 120F may comprise several such lines, parallel to each other. In some embodiments pattern 120F may span only part of the circumference of the stomach, or may be at an angle to the longitudinal axis of the stomach (rather than perpendicular to it).

Such a pattern may slow propagation through the gastric wall, of signals from the proximal part of stomach 101 to antrum 105 and pylorus 106.

In some embodiments, pattern 120F or similar patterns may be used to create a constricting ring or band around the middle of the stomach. Such a constriction may serve to impede flow of gastric contents towards the antrum, which may slow gastric emptying, increase satiety, and reduce obesity.

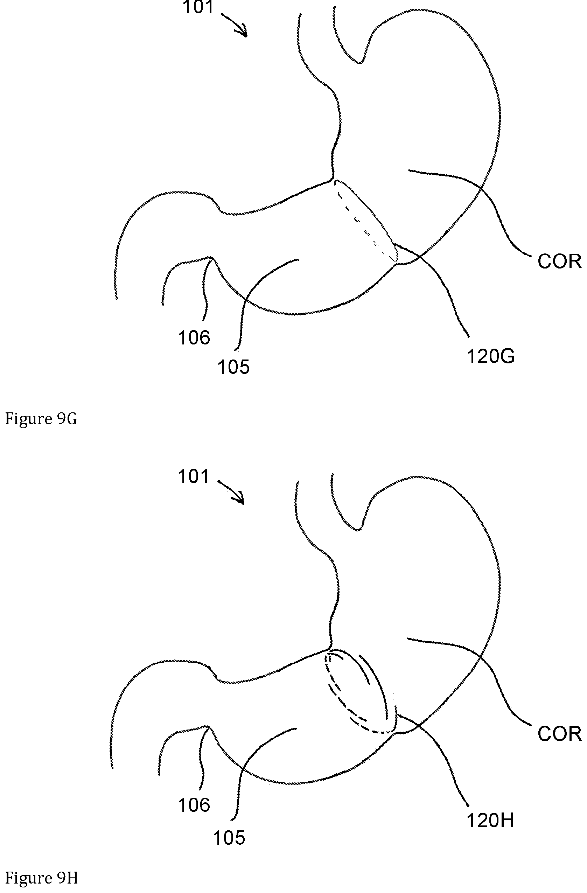

The "ring" or "band" constriction which may result from the ablation of a pattern 120G or similar, which may be intended to cause gastric tissue shrinkage, is depicted in FIG. 9G. The pattern may be a complete circle comprising a substantially continuous line as in FIG. 9G, or it may comprise an open circle or several overlapping segments resulting with a segmented circumferential constriction ring pattern 120H, as shown in FIG. 9H. Such a pattern may prevent excessive constriction and may still allow gastric pacemaker signals to pass, albeit at a slower pace. Various other constriction patterns may be possible including but not limited to wavy or zig zag circumferential lines.

Additional embodiments may include different global patterns such as zig zag lines instead of the spiral lines in pattern 120A, or the straight lines in patterns 120B-120F, point ablations (for example at any of the line intersections in FIG. 9D), or any combination of the above.

### Regional ablation patterns

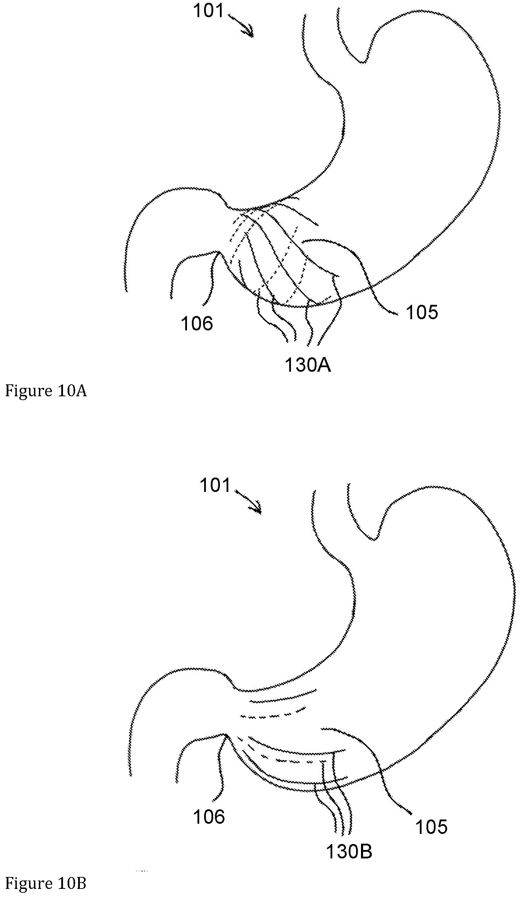

In some embodiments, depicted in FIGS. 10A-10D, the ablation patterns may be regional. The meaning of "regional" here is that the pattern may be dispersed over substantially most of a specific region of the stomach. Typically, such regions may include but are not limited to antrum 105, pylorus 106, and corpus COR of stomach 101.

Of note, the patterns may typically comprise thin lines, relative to the gaps between the lines, such that although the lesions extend over most of the treated region, most of the surface of this region may remain free of lesions, i.e. unablated.

Examples of such ablation patterns are shown in FIGS. 10A-10D in a stomach, although the treatment may be applied to many other organs. As will be explained below, the "regional" patterns may extend over substantially all the treated region, or at least over a large part of it.

More particularly, FIG. 10A is a schematic anterior view of a stomach 101, with an ablation pattern 130A ablated on the wall of stomach 1 at the region of antrum 5. Pattern 30A comprises several substantially continuous spiral lines extending from the proximal to the distal end of antrum 105. The lines may be substantially parallel to each other. In some embodiments, pattern 130A may comprise only one such line.

Such a pattern may slow both axial and circumferential propagation through the wall of antrum 105, which may reduce the rate of gastric emptying.

FIG. 10B is a schematic anterior view of a stomach 101, with an ablation pattern 130B ablated on the wall of stomach 101 at the region of antrum 105. Pattern 130B may comprise several substantially continuous lines, parallel to the longitudinal axis of the stomach, extending from the proximal to the distal end of antrum 105.

Such a pattern may slow circumferential propagation through the wall of antrum 105, which may reduce the efficacy of peristaltic contractions, and prolong gastric emptying time.

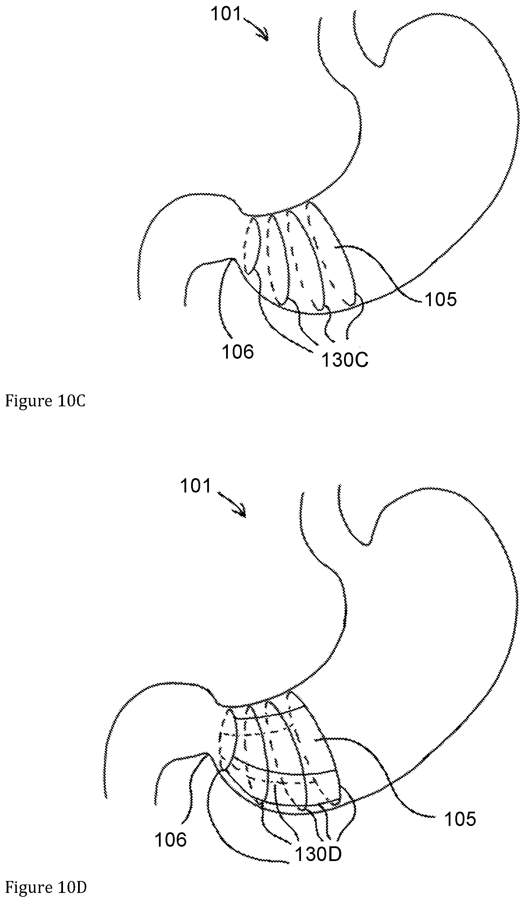

FIG. 10C is a schematic anterior view of a stomach 101, with an ablation pattern 130C ablated on the wall of stomach 101 at the region of antrum 105. Pattern 130C may comprise several substantially continuous lines, perpendicular to the longitudinal axis of the stomach, and optionally spanning the whole circumference of the stomach, extending from the proximal to the distal end of antrum 105.

Such a pattern may slow axial propagation through the wall of antrum 105, which may reduce the rate of gastric emptying.

FIG. 10D is a schematic anterior view of a stomach 101, with an ablation pattern 130D ablated on the wall of stomach 101 at the region of antrum 105. Pattern 130D is essentially a combination of patterns 130B and 130C, thus creating a grid of longitudinal and perpendicular lines.

Such a pattern may slow both axial and circumferential propagation through the wall of antrum 105, which may reduce the rate of gastric emptying.

### Local ablation patterns

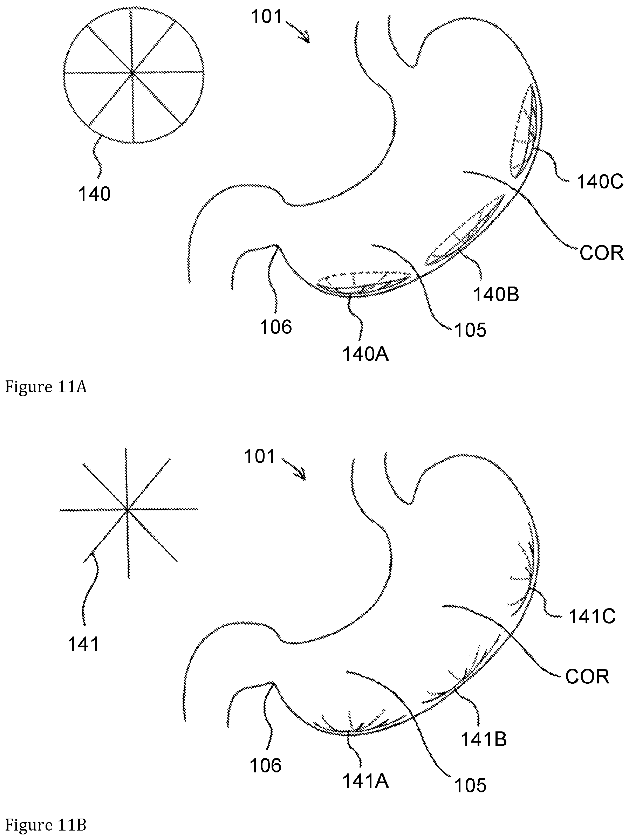

In some embodiments, depicted in FIGS. 11A-11D, the ablation patterns may be local. The meaning of "local" here is that the pattern may be created at a specific location, typically with a small area within stomach 101. Typically, such locations may include but are not limited to the inferior aspect of antrum 105, the middle of the greater curve 108, and the proximal end of the corpus COR.

Of note, the patterns may typically comprise thin lines, relative to the gaps between the lines, such that although the lesions extend over most of the treated location, most of the surface of this location may remain free of lesions, i.e. unablated.

Examples of such ablation patterns are shown in FIGS. 11A-11D in a stomach, although the treatment may be applied to many other organs.

More particularly, FIG. 11A is a schematic anterior view of a stomach 101, showing an ablation pattern 140 ablated on the wall of stomach 101, located by way of example at the following locations: 140A - at the inferior aspect of antrum 105, 140B - at the middle of the greater curve 108, and 140C - at the proximal end of the corpus COR. although shown here in these locations, pattern 140 may be ablated at any location in the stomach.

Pattern 140 comprises eight lines extending radially from a central point, and a circular line around them. The lines may be substantially continuous, and may be at equal distances from each other. In some embodiments, pattern 140 may comprise any number of radial lines, typically 3-12 lines. In some embodiments, pattern 140 may comprise the circular line only.

Such a pattern may slow or prevent propagation from the treated location.

FIG. 11B is a schematic anterior view of a stomach 101, showing an ablation pattern 141 ablated on the wall of stomach 101, located by way of example at the following locations: 141A - at the inferior aspect of antrum 105, 141B - at the middle of the greater curve 108, and 141C - at the proximal end of the corpus COR. although shown here in these locations, pattern 141 may be ablated at any location in the stomach.

Pattern 141 comprises eight lines extending radially from a central point. The lines may be substantially continuous, and may be at equal distances from each other. In some embodiments, pattern 141 may comprise any number of radial lines, typically 3-12 lines.

Such a pattern may slow or prevent propagation from the treated location.

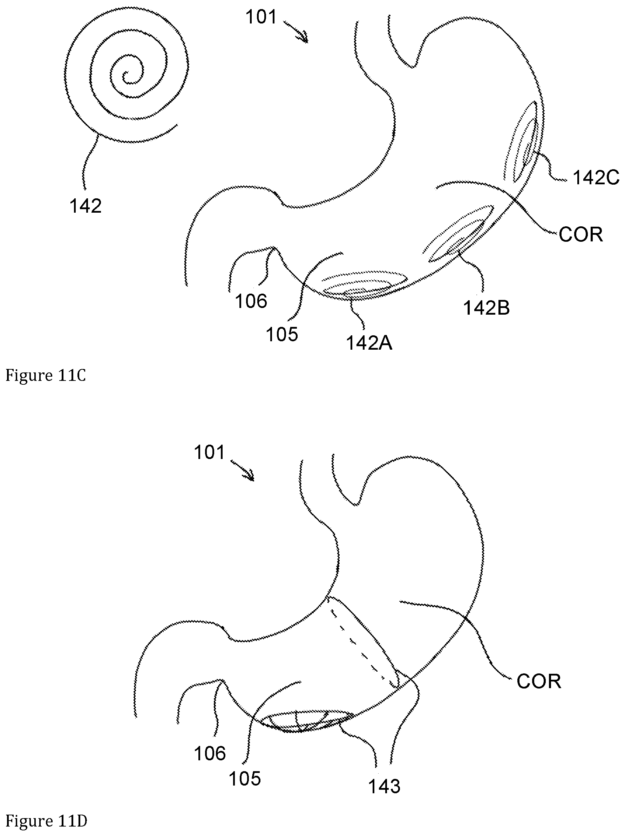

FIG. 11C is a schematic anterior view of a stomach 101, showing an ablation pattern 142 ablated on the wall of stomach 101, located by way of example at the following locations: 142A - at the inferior aspect of antrum 105, 142B - at the middle of the greater curve 108, and 142C - at the proximal end of the corpus COR. although shown here in these locations, pattern 142 may be ablated at any location in the stomach.

Pattern 142 comprises a spiral line extending from a central point, creating a flat spiral at the treated location. The line may be substantially continuous. In some embodiments, pattern 142 may comprise several such lines, substantially parallel to each other.

Such a pattern may slow or prevent propagation from the treated location.

### Other ablation patterns

FIG. 11D is a schematic anterior view of a stomach 101, showing an ablation pattern 143 ablated on the wall of stomach 101.

Pattern 143 is essentially a combination of a "local" pattern 140A and a "global" pattern 120F. Although shown here in these locations, pattern 143 may be ablated at any location in the stomach.

Such a pattern may slow or prevent propagation of signals from the treated location.

In some embodiments, any combination of the patterns and locations described herein may be used.

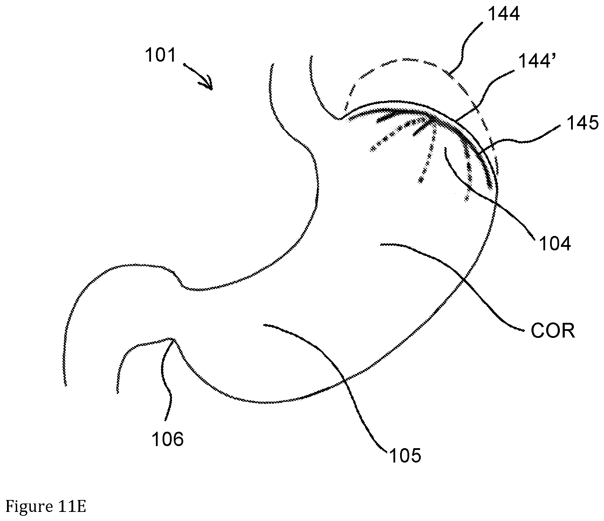

In some embodiments, the goal of treatment may alternatively or additionally be to reduce the volume and/or distensibility of at least a portion of the stomach. The fundus 104 of the stomach is normally very distensible, and is where food normally accumulates during meals. Reducing the volume and/or distensibility of the fundus may therefore promote early satiety and alleviate obesity. Such a therapy may be used instead of, or as an adjunct to bariatric surgery including gastric bypass, placement of gastric bands, etc. It may be delivered at fundus 4 as depicted in FIG. 11E, or at other areas of the stomach.

More particularly, FIG. 11E is a schematic anterior view of a stomach 101, with an ablation pattern 145 ablated on the wall of stomach 101 at the region of fundus 104. Dashed line 144 marks a possible pre-treatment contour of fundus 104, whereas line 144' marks a possible post-treatment contour of fundus 104.

Although the specific pattern 145 shown here is similar to local ablation pattern 141, any pattern that may reduce the volume of the fundus may provide the desired effect. Thus, for example other patterns, such as, but not limited to patterns 130A-130D and 140-142 described herein, may be used in the fundus or other relevant gastric areas to reduce their volume and/or compliance.

### Lesion depth

The lesions making up the above described ablation patterns may be of various depths and may include several of the gastric wall layers shown in FIG. 8B.

Typically, lesions may extend through the mucosa MCS, submucosa SMC, and at least part of the muscularis MUS. In some embodiments, the lesions may be completely transmural, reaching the serosa SER, however visceral peritoneum 116 may typically be avoided, so as not to risk extension of the lesion to adjacent organs.

In some embodiments, skipping of mucosa MCS or at least the outermost layers of mucosa MCS, or reduction of the extent of thermal damage to those layers may be achieved by cooling those layers as known in the art. This may be done for example by cooling of the contents of the stomach or organ prior to or during the procedure, or by application of a continuous infusion of cooled fluid at the ablation sites, optionally through the ablation device itself.

Typically, a longer ablation time and total energy may need to be used in order to induce a tissue contraction as opposed to just limiting propagation of signals in the organ wall. The normal adult human gastric wall is 4-6mm thick. Typical depths of lesions for affecting signal propagation may be 0.2-4mm, preferably 0.5-2mm. Typical depths of lesions for inducing tissue shrinkage and constriction may be 2-6mm, preferably 3-5mm.

Control of the depth of ablation may be achieved by several methods. In case of bipolar electrodes as described herein, the distance between the two poles affects the depth of ablation at any given power, and may therefore be pre-determined and constructed to produce the desired depth. The power, duration, and energy density of the delivered treatment may also be modified to control the depth of the lesions.

The temperature of the tissue may be measured by thermistors and used to stop ablation once temperature reaches a predetermined threshold.

### Effects of ablation patterns:

As previously stated, the goal of creating the above lesion patterns in the stomach or organ wall, may be to modify propagation of signals through the wall in order to affect a change in behavior of the organ as a whole.

This may for example be achieved by a partial block of the connection between ICCs. Alternatively this may be achieved by a complete block of contacts between ICCs.

A partial or complete block of conduction between neurons within the Submucosal Plexus SP and / or Myenteric Plexus MP may constitute another mechanism by which the effects of treatment may be achieved.

Direct propagation between muscle cells is not considered a significant pathway of signal conduction in the gut, however blocking of such conduction by the lesions of the current treatment may also contribute to modifying peristalsis.

The above may for example be achieved by delivering energy sufficient to create a scar within the tissue. Typically, such a scar may develop over time during the healing process following the ablation, and may involve deposition of collagen. Gentle lesions causing minimal, thin scarring, may be sufficient to block propagation of signals in the gastrointestinal wall. Wider and deeper lesions may additionally or alternatively cause more extensive scarring, which may affect the mechanical properties of the ablated organ.

Thus, another mechanism which may contribute to treatment of obesity is a potential decrease of gastric volume by creating lesions sufficient to cause its contraction, or a decrease in its elasticity.

In some embodiments of the current invention, the goal of the treatment may be modulation of the natural propagation of intrinsic signals within the stomach with the objective of opposing the known effects of such intrinsic activity. For example, the intrinsic activity is known to maintain smooth muscle relaxation. Thus, disturbing this activity may cause an increase in stomach tone that may promote early satiety (when the stomach resting tone is increased to a degree of certain rigidity).

For example, the intrinsic activity is known to mediate sphincter relaxation. Thus, disturbing this activity may cause an increase in sphincter tone that may promote early satiety (by delaying gastric emptying) and/or alleviate reflux (by increasing gastro esophageal junction/sphincter tone).

In another example - intrinsic muscle activity is known to have an important role in stirring the gastric contents, and improving digestion. Disturbing such stirring may result in more of the gastric content being only partially digested, which may cause prolonged gastric emptying times, increased satiety and reduced food intake.

In another example - intrinsic gastric activity is known to cause "wavy" movements of the gastric serosa, thus optimally exposing (unfolding) all gastric crypts. Disturbing such activity may allow eroded gastric mucosa to remain less exposed to the gastric content, thus alleviating erosive gastritis.

In yet another example, coordinated gastric relaxation, mediated by the internal gastric conduction system, normally allows the stomach to rapidly increase in volume to accommodate large meals. Disruption of such coordinated relaxation sequences may hinder the ability to consume large amounts of food in a short time (help prevent binge eating).

In another example, the coordinated gastric activity normally generates a strong pacemaker signal that can affect the entire GI tract. Disrupting such activity may reduce the amplitude of gastric activity, thus reducing the excitation of the entire GI, alleviating symptoms of irritable bowel syndrome.

In another example, when the stomach is empty for a prolonged period of time, intrinsic activities cause gastric contraction which minimizes the gastric volume and the sensation of hunger. Disrupting such activity may reduce the ability of the stomach to inhibit hunger, thus enabling treatment of anorexia nervosa, and anorexia of other causes.

In another example - in case of digestion of toxin or bacteria laden food (or other obnoxious stimuli), multiple foci generate widespread non-coordinated gastric activity resulting in a functional paralysis of the stomach, accompanied by an acute sensation of nausea. By limiting the ability of each focus to reach the entire stomach, nausea of various causes may be alleviated.

In another example, the coordinated contraction of the stomach (mediated by the intrinsic conduction system) is important to increase gastric pressure to propel food out of the stomach and into the duodenum and/or the esophagus (in case the esophageal sphincter pressure is lower than the pyloric pressure). Disturbing such coordinated contraction may attenuate increases in gastric pressure, alleviating reflux symptoms, delaying gastric emptying, reducing belching and bloating and more.

In yet another example, coordinated gastric activity is required for vomiting. Disturbing such intrinsic conduction may inhibit the ability to vomit, as a potential treatment in cases of bulimia and/or cyclic vomiting.

In another example, the refractory period after a contraction is an important mechanism that helps to prevent "re-entry" circles of self-perpetuating contractions. When a certain stomach zone contracts, it becomes refractory, thus preventing the same contraction to now travel back to its origin. By isolating parts of the stomach, re-entry circles may become possible, thus disturbing the normal activity of the stomach and potentially causing continuous rapid contractions of the stomach. Such contractions may reduce gastric emptying, induce a lack of appetite, and even travel along the GI tract to increase intestinal motility and alleviate constipation and/or ileus.

In an embodiment, a narrow isolated tissue strip may be created, leading from the body of the stomach to the fundus of the stomach. The strip may have two openings on both ends of the strip. The strip may be designed in the shape of a "winding road", so that propagation along the tissue strip takes longer that the propagation in adjacent tissue (where propagation may occur along straight lines). Thus, a propagating signal traveling form the body to the fundus may advance in two ways: either directly, or through the strip. The propagating signal traveling through the strip may arrive at the fundus later than signals traveling directly (through the unmodified gastric tissue). If the delay is long enough, the delayed signal may reach the fundus after it has already contracted and already completed the refractory period, re-exciting the fundus in a "re-entry" mechanism. Typically, in order to incite re-entry, the tissue strip must have a linear length (minimal length of conduction through the strip) that is at least two times that of the parallel path, and at least 10cm in length.

### Non-ablative applications

The above described novel bi-polar wire electrode may also be used to treat GI disorders by delivering electrical currents to stimulate GI smooth muscle contractions. Due to the flexible nature of the novel wire electrode an electrode, array can cover a large surface area while remaining easy to insert into the GI tract, easy to pass through the GI tract, and even easy to swallow with comfort.

Such a multi bipolar electrode array may be used to deliver electrical currents to contract GI smooth muscle, in order to "train" the muscle and eventually modify its structure and activity. In one example, an electrode array may deliver stimulation to the distal esophagus, to tighten the esophageal sphincter and increase its tone, to treat gastroesophageal reflux. This array may be advanced into the esophagus on a semi-rigid tube, or alternatively it may be swallowed by the patient and held at the desired location by anchoring the proximal wire to the patient (for example, taping to the cheek, or anchoring to a tooth). In another example, such an electrode array may be inserted through the anus, expanded to contact the rectal wall and it then may be used to stimulate the rectum to increase rectal tone, to treat constipation and/or soiling.

Such stimulation therapies usually necessitate repetitive treatment sessions. Since both conditions are not life threatening a viable therapeutic solution must be extremely comfortable to use repetitively. The novel bi-polar electrode allows electrode array designs that can expand to contact large surface areas.

Bi-Polar nitinol wire electrode - Another possible application is to couple the novel wire electrode to a nitinol wire. When current is applied through the electrode wire, the nitinol is activated to assume a structure that approximates the electrodes to the desired surface/material.

### Energy types

Although described below using electrical energy at Radio-Frequency (RF), any modality and type of energy known in the art to be used for therapeutic ablation of tissue, may be used for the purpose of creating the ablation patterns described herein. These modalities may include but are not limited to microwave or high frequency electrical ablation, irreversible electroporation, ultrasound, cryoablation, laser, mechanical scoring, and more. The conductors of the devices described herein may typically be substituted for appropriate ablation elements compatible with the other modalities, for example - cooled conductors or tubes for passage of cooled gas for cryoablation, optic fibers for laser, etc.

### Devices

Following are descriptions of embodiments of devices intended to create the ablation patterns mentioned above.

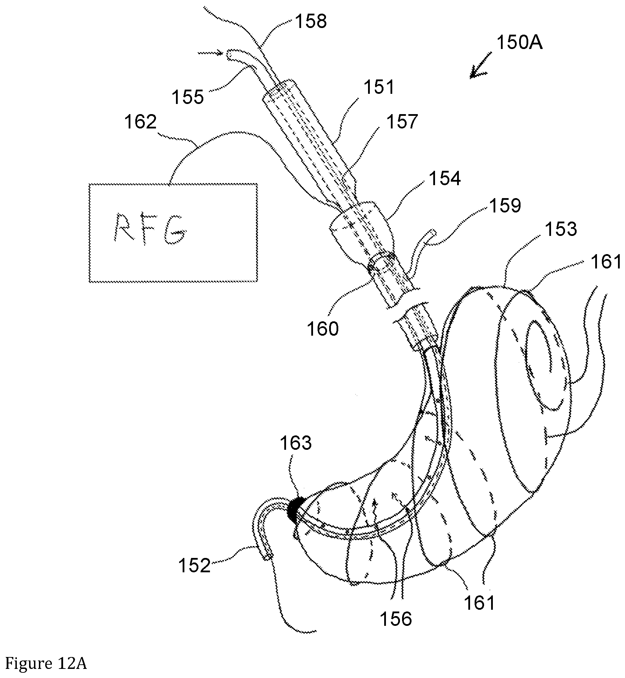

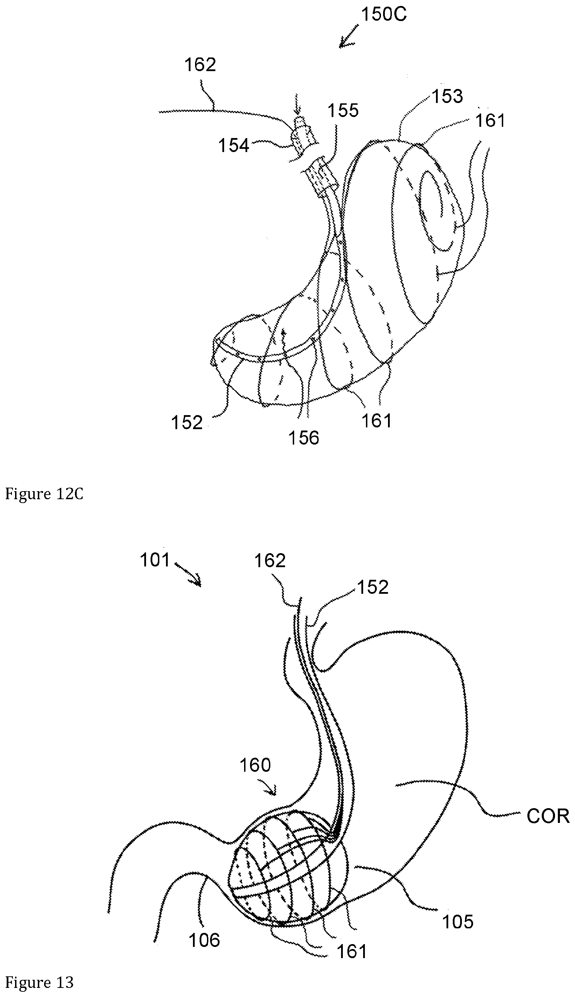

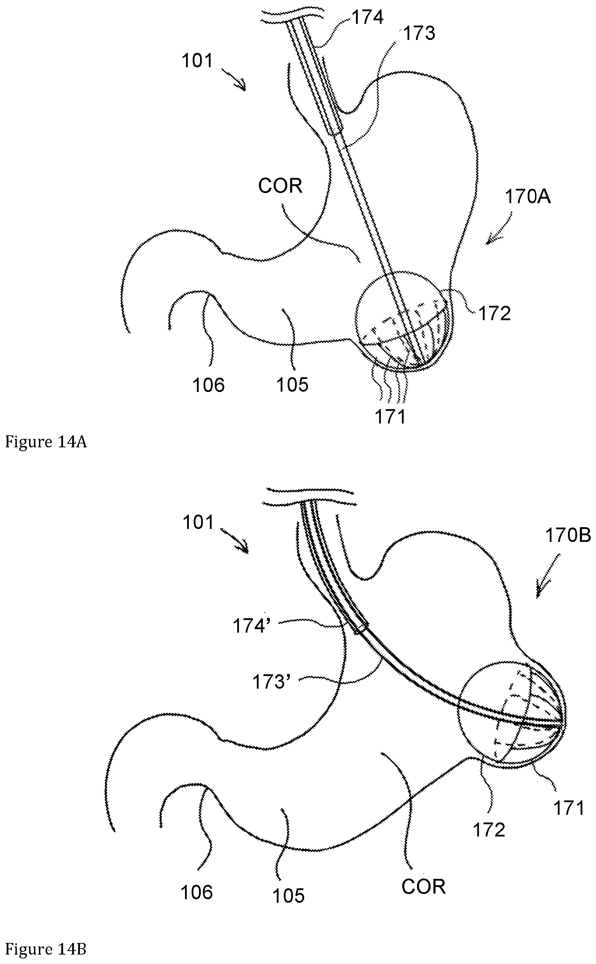

FIGS. 12A-12C describe devices intended for creating global ablation patterns, FIG. 13 is an example of a device for creating regional ablation patterns, and FIGS. 14A-14B describe devices intended for creating local ablation patterns.

More particularly, FIG. 12A is a schematic frontal view of device 150A which may be used to create a global ablation pattern in a human stomach, shown here in its fully expanded state, outside the body. Device 150A may comprise conductors (or ablation elements) 161 mounted over an expandable member 153 which may typically be an inflatable balloon, but may also be any other appropriate expandable member as known in the art, such as a wire cage. Expandable member 153 may be mounted over a flexible shaft 152 which may pass through sheath 154, with seal 164 between them. Sheath port 159 may be in fluid communication with the lumen of the treated organ, through the lumen of sheath 154. Shaft 152 may comprise a handle 151 at its proximal end. An expansion port and lumen 155 may provide access for inflation of expandable member 153 through openings 156 in shaft 152 in case of a balloon, or for a cable or other element for expansion, in case of a different form of expandable member. An optional lumen 157 extending through shaft 152 may allow introduction of device 150A into a patient's body over a guidewire 158. Optional tip 163 may be located at the distal end of expandable member 153, securing it to shaft 152. Conductors 161 may be connected via cable 162 to RF generator RFG, or other energy source as appropriate.

As seen in FIG. 12A, expandable member 153, in the current embodiment comprising an inflatable balloon, may have a shape compatible with the inner volume of a typical stomach. Balloon 153 may for example be made of a relatively or completely noncompliant material e.g. high durometer polyurethane, or PEBAX, so as to achieve a defined and stable shape and volume upon complete inflation. In such case, balloons of various sizes may be used to fit different patients. Alternatively, balloon 153 may be manufactured of a compliant material such as latex or low durometer polyurethane, and inflated to the appropriate size for each specific patient.

Inflation may typically be performed using air, but may also be done using other gases, or fluid such as saline.

Expansion of expandable member 153 to a volume larger than that of the empty stomach may be beneficial, for example by flattening or eliminating the gastric folds (rugae), thus improving contact between conductors 161 and the gastric wall.

Conductors 161 may typically be any type of ablation element capable of transmitting the required energy to the organ wall to create the desired ablation patterns. Monopolar electrodes may be used, in which case they may typically be used with a dispersive/patient /ground electrode elsewhere on the patient's body, e.g. on the patient's thigh or back.

Conductors 161 may be made of multiple electrically isolated segments, that may be activated in succession or synchronously.

Bipolar electrodes may be used for conductors 161. Such bipolar electrodes may for example be two parallel conductors at a constant distance from each other. In some preferred embodiments, spiral bipolar electrodes, may be used. Again, a single pair of such spiral bipolar electrode, or several separate segments thereof, may be used.

Conductors 161 may typically be connected to expandable member 153 using methods known in the art, including adhesion, use of clips, threading through loops in the balloon, etc.

Conductors 161 may further be slideable over the balloon, and pulled into their final position by inflation of the balloon.

The dimensions of flexible shaft 152 may typically be:
Length: typically 20cm-120cm; preferably 40cm-100cm
Outer Diameter: typically 2mm-30mm; preferably 5mm-15mm

The dimensions of sheath 154 may typically be:
Length: typically 10cm-90cm; preferably 30cm-60cm
Outer Diameter: typically 4mm-40mm; preferably 6mm-25mm

Although device 150A is appropriate and designed for use without visualization, in some embodiments shaft 152 further comprises or alternatively allows for insertion of means for visualization such as fiber optic means or a digital camera, typically with appropriate means of illumination for viewing the inside of the treated organ prior to or during the ablation procedure. Such visualization means may enable viewing of the lumen of the organ through expandable member 153, for example by expandable member 153 being a transparent balloon, and such camera being located in the middle of expandable member 153.

Device 150A is shown here with conductors 161 arranged spirally around expandable member 153, a version appropriate for creating ablation pattern 120A shown in FIG. 9A. Different arrangement of conductors 161, compatible with patterns 120B-130D, and 141-143, may be used to create any of those ablation patterns shown in FIGS. 9B-11E.

In use, following performance of a gastroscopy, a guidewire may be left in the stomach, extending out of pylorus 106 into the duodenum. Device 150A may be inserted over guidewire 158, which may help to guide it such that the distal end of shaft 152 enters the duodenum, and tip 163 is positioned at pylorus 106. Insertion of device 150A may be performed in its crimped state, i.e. with sheath 154 covering shaft 152 and expandable member 153 all the way to tip 163.

Following insertion of shaft to the desired depth, sheath 154 may be retracted to expose expandable member 153 and conductors 161. Expandable member 153 may then be expanded until it fills the gastric cavity and conductors 161 may come into contact with the gastric wall. Suction through sheath port 155 may be used to remove any residual fluids between the device and gastric wall. Contact of conductors 161 with the stomach wall may be verified as will be detailed below. Ablation may be performed by delivering energy from RFG to conductors 161.

Following ablation, the stomach may be inflated with fluid or air to assist removal of device 150A. Expandable member 153 may be collapsed or deflated, and sheath 154 may then be pushed forward to cover expandable member 153 and conductors 161. Device 150A may then be retracted and removed from the patient's body.

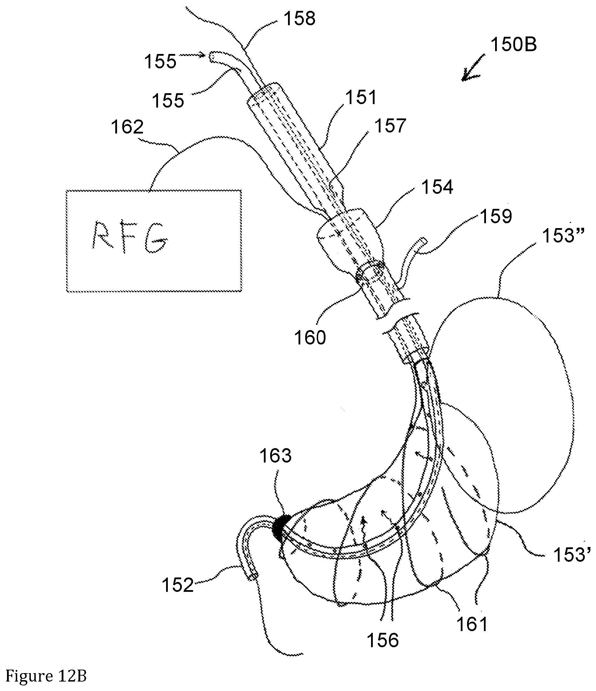

FIG. 12B shows device 150B which is a variation of device 150A in which instead of a single balloon 153, two balloons 153' and 153'' may be used, which may make deployment and retraction of the device easier. In some embodiments, balloon 153'', which may fill the fundus to provide a counter force to balloon 153' and stabilize it in place, may be inserted into the stomach separately, for example prior to insertion of device 150B.

FIG. 12C shows device 150C which is a variation of device 150A in which shaft 152 may not extend beyond the distal tip of balloon 53, and a guidewire lumen may be absent. This embodiment may be inserted "blindly" or using fluoroscopy, or ultrasound guidance, and proper positioning of the device may depend on inflation of balloon 153 to match the shape of the stomach.

FIG. 13 shows a device for creation of the regional ablation pattern 130C, shown in FIG. 10C.

More particularly, FIG. 13 is a schematic frontal view of stomach 101 with device 160 in it, shown in its fully deployed state within antrum 106.

Device 160 may be generally similar to device 150C, however it may comprise a smaller expandable member, so that it fits within antrum 106. Shaft 152 may be flexible enough to allow bending so that the tip of shaft 152 may reach pylorus 106, and rigid enough to allow control of its position.

With modification of the arrangement of conductors 161, device 160 may be used to create regional ablation patterns 130A-130D shown in FIGS. 10A-10D.

Device 160 may be used to place the same patterns in other regions of the stomach as well.

FIG. 14A shows a device for creation of the local ablation pattern 140B shown in FIG. 11A.

More particularly, FIG. 14A is a schematic frontal view of stomach 101 with device 170A in it, shown in its fully deployed state depressed against greater curve 108.

Device 170A may be similar to a device previously described in PCT application IB2017/000783 filed June 6, 2017. It comprises an inflatable balloon 172 mounted over a rigid, substantially straight shaft 173 and sheath 174. The main difference may be that conductors 171 may be the same as described for devices 150A-160 above, preferably using the bipolar electrodes described herein.

A different version of device 170A adapted for creation of the local ablation patterns 140A and 140C shown in FIG. 11A, is shown in FIG. 14B.

More particularly, FIG. 14B is a schematic frontal view of stomach 101 with device 170B in it, shown in its fully deployed state depressed against the proximal end of greater curve 108.

Shaft 173' and sheath 174' of device 170B may be rigid or semi rigid, but both may be curved at the same radius, which enables device 70B to reach more proximal or more distal areas of stomach 101.

In some embodiments, shaft 173' may be configured to be actively curved following insertion into the stomach, such that treatment may be delivered to more proximal areas, such as fundus 104.

In some embodiments, device 170B or similar to it may be used to deliver the fundus contracting therapy described in FIG. 11E.

In some embodiments, conductors 161 may be embedded or printed over balloon 153 of any of the above embodiments.

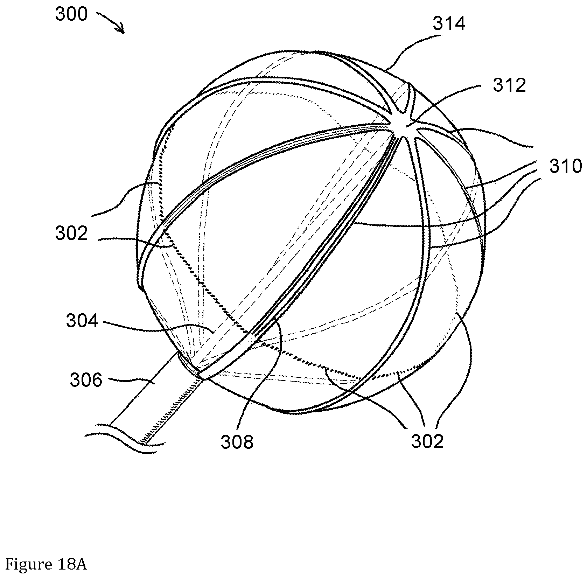

In some embodiments, the flexible bipolar electrodes described herein may be incorporated in a single device together with other electrodes of different types. In some embodiments, the other electrodes may be monopolar electrodes that may be coupled with a distant dispersive patient electrode. In some embodiments, the other electrodes may be bipolar, coupled either with a distant patient electrode, or with other bipolar electrodes on the same device. In some of these embodiments, the additional coupled bipolar electrodes on the device may be far from each other. In yet another embodiment, the additional coupled electrodes may be at a short distance from each other. A specific embodiment of such a device incorporating spiral bipolar electrodes together with parallel near-field bipolar electrodes is described in figures 18A-C. The benefit of using this combination of electrodes is that the parallel electrodes lend themselves to manufacturing as part of a PCB (Printed Circuit Board) but cannot fold in a direction perpendicular to their longitudinal axis, while the flexible spiral bipolar electrodes may be easily folded in any direction, thus enabling creation of lesions perpendicular to the longitudinal ones, which may be desirable in various situations.

More particularly, FIG. 18A is a simplified three-dimensional depiction of tissue ablation device 300 at its deployed state. Depicted are (from the left in a counter-clockwise direction) spiral bipolar electrodes 302, inner shaft 304, external sheath 306, parallel (or "rail track") bipolar electrodes (only one of these is annotated, for clarity), longitudinal PCB legs 310 (in this embodiment there are 8 legs, only 4 are annotated for clarity), distal tip 312, and expandable element 314.

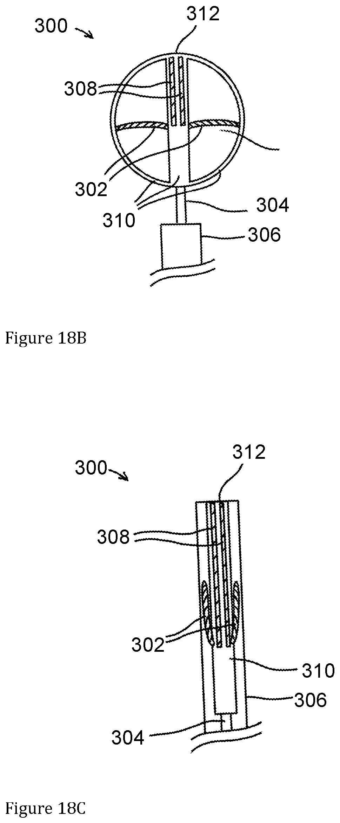

Typically, longitudinal PCB legs 310 may be slidably positioned over expandable element 314 (in the current image a balloon), which in turn may be mounted over inner shaft 304, which may be slidably positioned within external sheath 306. Flexible spiral bipolar electrodes 302, which may for example be any of the electrodes described above in FIGS. 1-6, may be attached to longitudinal PCB legs 310, such that when expandable element 314 is fully expanded, electrodes 302 may straighten out, and when element 314 is collapsed, electrodes 302 may fold, as shown in figure 18C. Parallel electrodes (or "rail track" electrodes) 308 are elongate conductors, at least two on each PCB leg 310 that may be positioned at a small distance from each other, typically 0. 1mm-2mm. When energized, current may flow between the two parallel conductors, affecting the tissue between them, along their length.

FIGS. 18B and 18C are simplified schematic side views of device 300 in its deployed and crimped states respectively.

More particularly, 18B shows device 300 at its deployed state. In this schematic illustration only 4 PCB longitudinal legs 310 are shown. The complete structure comprised of the electrodes and balloon is seen protruding out of external sheath 306 and ready for ablating tissue.

FIG. 18C shows device 300 at its crimped state. The complete structure comprised of the electrodes and balloon is seen folded inside external sheath 306.

In use, device 300 may be inserted into a patient's body via an orifice at its crimped state, and it may then be deployed to so that the electrodes may be in contact with the inner walls of a hollow organ. Following ablation external sheath 306 may be advanced distally over the device to crimp it and enable removal from the patient's body.

### Procedure

The devices and procedures described herein may typically be applied endoscopically. In some embodiments, insertion of devices into the target organs may be performed surgically. In some embodiments, a combined surgical and endoscopic approach may be used, wherein some aspects of the procedure may be performed endoscopically, and others surgically.

In some embodiments, such as those shown in FIGS. 12A-12C, treatment may typically be delivered at once, without moving the device during the procedure. In some embodiments, for example those shown in FIGS. 13-14B, several energy deliveries may be performed, each with the device at a different position, to achieve the complete ablation pattern required.

In some embodiments, various forms of mapping of gastric activity may be performed prior to ablation in order to determine the pattern of activity in the stomach, including for example location of pacemaker, conduction pathways, any aberrant sources of activity, conduction velocity, or gastric emptying velocity.

This may for example be accomplished by using real time video analysis of endoscopic clips of the inside of the stomach, to image gastric motility.

This may also optionally be performed using high definition ultrasound of the stomach wall.

Another possible way of analyzing gastric activity is using electrode arrays endoscopically inserted into the stomach or placed surgically on its outer surface.

By employing such methods, determination of aberrant sources of gastric activity, or exact localization of the gastric pacemaker may be used to plan the appropriate lesion patterns.

As will be detailed below, in some embodiments, the data collected using any of the above methods may be saved into a database together with other relevant clinical information of the patient, so that correlations may be sought between gastric activity patterns and their clinical manifestations.

In some embodiments, the above acquired data may be used to create computerized simulations of the current gastric activity, as well as of the anticipated effects of the intended interventions on target organ activity and/or or structure, to enable planning of the appropriate ablation pattern.

### Ensuring proper device fit

Good contact between the electrodes or other ablation elements and the organ wall may be crucial for achieving good ablation results. Various approaches may be used to ensure good contact despite variability in the anatomy of target organs between different patients.

In some embodiments, ablation devices of several sizes may be available, and the appropriate size may be chosen by the user. Choosing the device may be based on measurements made endoscopically or using imaging, or by use of a sizing device. Such sizing device may for example be a balloon which may be inserted into the target organ prior to the ablation device, and may be inflated to a known pressure or volume. Once inflated, verification of fit may be done using imaging, endoscopy, measurement of impedances between electrodes on the surface of such a device, or any other method as known in the art.

For example, a sizing device may comprise multiple electrical contacts, shaped as points, strips, or other shapes, distributed over its surface. Measurement of impedances between the electrodes and / or between the electrodes and a reference electrode inside or outside the patient's body, may be performed. In some embodiments, such measurements may be done simultaneously during inflation of the sizing device balloon. By plotting impedances against inflated volume and/or device diameter at certain locations along the device, and/or other device dimensions, optimal device dimensions, that may be defined as those at which the best contact is measured, may be identified, in real time or after the procedure. An appropriate ablation device size, inflation volume, and/or shape may be chosen accordingly.

In some embodiments, such a sizing device may comprise a compliant balloon so that it can be used for all, or most patient sizes. In some embodiments, sizing devices of different sizes, that comprise non-compliant balloons, may each fit only a specific organ size or range of sizes, and several sizes of such sizing devices may be available to choose from. In some embodiments, the sizing device may comprise a transparent balloon, and either enable insertion of an endoscope into the balloon, or it may comprise a camera inside the balloon, so that visual confirmation of good wall contact may be done through the balloon.

In some embodiments, the ablation device may also function as the sizing device. In some embodiments, an ablation device of the "one-size-fits-all" type may comprise a compliant balloon and sliding electrodes, which may be pulled in by the inflating balloon until the balloon fills the organ snugly, and the electrodes may then be in good contact with the organ wall.

Determination of proper contact between conductors 161 and the gastric wall may be done using any of measurement of impedance through conductors 161, by measuring temperature near conductors 161, or by use of imaging, including fluoroscopy, or ultrasound. In some embodiments, stimulation of the gastric wall by delivering a current through conductors 161 may be done, while measuring the effect on heart rate or its variability, a significant effect may be evidence of good contact.

### Colon treatment for constipation

While the above devices may be used for treating the stomach as well as other gastrointestinal organs for various conditions, specific embodiments of such devices, especially suited for treating constipation, will be described next.

Constipation is a huge medical problem, affecting approximately 80 million people in the US alone.

Of particular interest is constipation that develops as a consequence of age related changes in the colon.

Studies in humans have shown that aging is associated with excessive relaxation of the ascending colon, probably caused by decreased cholinergic function in this area *(*Changes in neuromuscular structure and functions of human colon during ageing are region-dependent. Broad et al., Gut 2018;0:1-14*.).*

Similar changes probably take place in the cecum as well, which according to clinical observations, demonstrates age related dilatation. Such relaxation and dilatation may lead to reduced propulsion of stool, which may result in constipation and even fecal impaction.

Reducing the volume of the cecum and right (ascending) colon, and/or decreasing their compliance, may improve movement of stool through the large intestine, as a treatment for constipation. Another treatment modality contemplated here is ablation of the colonic mucosa to reduce water absorption within the colon.

The above may be achieved for example by producing ablation patterns that may result in any of scarring of the colonic wall, reduction of its volume, reduction of its capacity to store stool, or reduction of its capacity to absorb water.

In addition, the inventors believe that the dilatation and relaxation of the colon may, at least in part, be mediated by a rapid arrhythmia pattern of conduction within the colon, much like the fibrillation seen in the atria of the heart. In both cases dilatation is accompanied by loss of contraction force and loss of expected periodic activity. Partitioning of the colon (as described above for the stomach) may prevent such fibrillations of the colon and may allow normal colon function. Typical ablation patterns may create partitions of the colon, mostly isolated form the rest of the organ.

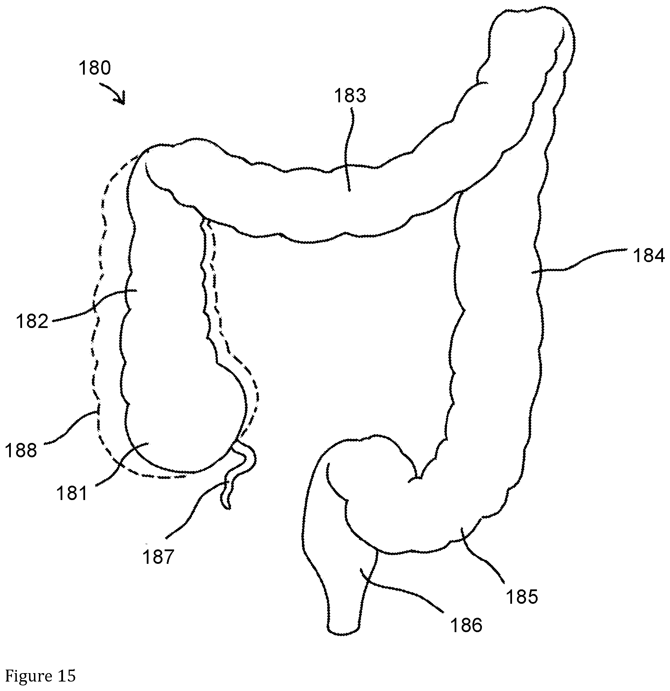

FIG. 15 is a simplified anterior view illustration of a human large intestine 180.

More particularly, starting from the left lower quadrant and moving clockwise are seen cecum 181, ascending (right) colon 182, transverse colon 183, descending (left) colon 184, sigmoid colon 185, and rectum 186. The appendix 187 is seen connected to cecum 181. Dashed line 188 marks a possible contour of a dilated cecum and ascending colon.

The normal adult human cecum and right colon wall thickness is 1.5 mm on average, with a range of roughly 1.2 mm to roughly 2 mm.

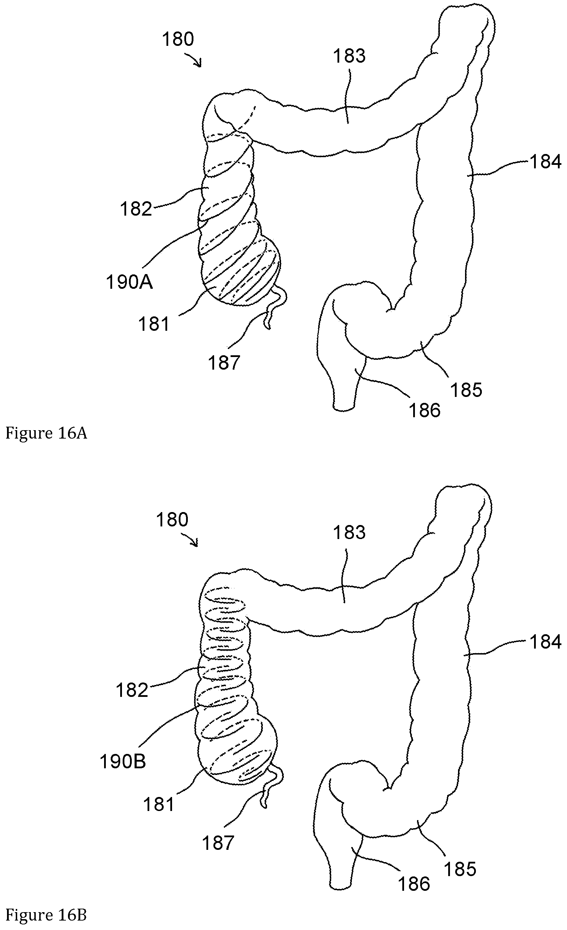

Some possible treatment ablation patterns are depicted in FIGS. 16A-16B. Of note, ablation patterns are shown here on a normally sized colon, as its diameter is expected to decrease following treatment.

More particularly, FIG. 16A is a simplified schematic anterior view of spiral ablation pattern 190A, produced in the wall of a human large colon.

Spiral ablation pattern 190A may typically extend from the cecum to the hepatic flexure (between ascending colon 182 and transverse colon 183). It may start anywhere in the cecum or right colon and may comprise a single line or multiple substantially parallel lines, at the same clockwise direction, or at opposite directions. Pattern 190A may have a constant pitch, i.e. the distance between each two adjacent loops of the spiral may be constant, or, as depicted in **FIG.** 16A, the pitch may vary, for example it may gradually or abruptly increase from cecum 181 towards transverse colon 183. Such a change in pitch may create a gradient in the compliance and/or diameter of the intestine along the treated segment, which may promote movement of stool towards the rectum.

In another embodiment depicted in FIG. 16B, a pattern of interlaced half circles along the cecum and ascending colon may be used.

More particularly, FIG. 16B is a simplified schematic anterior view of spiral ablation pattern 190B, produced in the wall of a human large colon.

As with pattern 190A, pattern 190B may typically extend from the cecum to the hepatic flexure, and may have a constant or variable pitch.

Patterns 190A and 190B may enable decreasing the volume of the intestine while preserving the contractility of at least the untreated tissue between the thin ablation lines, as well as the propagation of intrinsic activity within the intestinal wall. These patterns may also avoid excessive stricture of the intestine as the full circumference of the colon is not ablated at any one location.

With any ablation pattern used in the cecum, avoiding the ileo-cecal valve and opening of the appendix may be important for prevention of strictures, bowel obstruction, and appendicitis.

Avoiding these structures may be achieved for example by endoscopically identifying them and covering them with thermal shields, or by positioning the ablation device such that it does not ablate these locations.

Performance of cecal and or ascending colon ablation for creating ablation patterns such as 190A and 190B or similar, may be achieved by various devices.

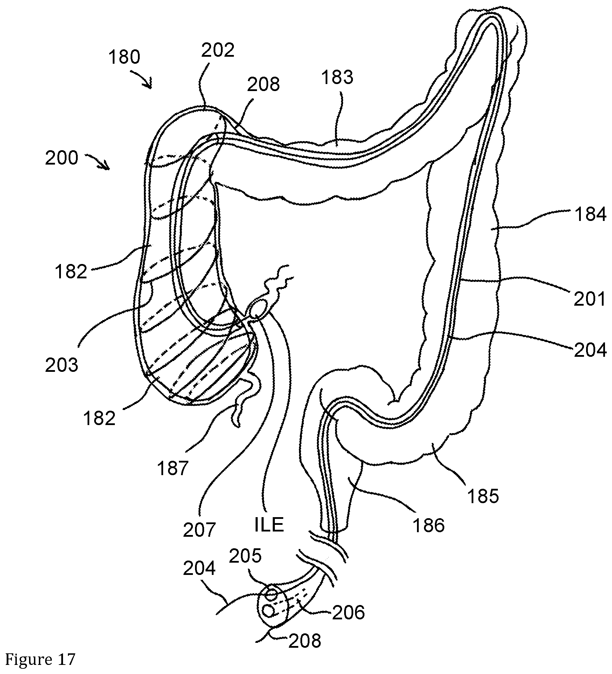

An example of an embodiment of a cecal and / or ascending colon ablation device is described in FIG. 17.

More particularly, FIG. 17 is a simplified schematic anterior view of ablation device 200 in large colon 180. The distal ileum ILE is seen connecting to cecum 181 adjacent appendix 187. Device 200, which may be generally similar to the devices described in FIGS. 12A-14B, comprises a flexible shaft 201, a balloon 202, an electrode or electrode array 203, a guiding element lumen 205, typically going from proximal to distal end of flexible shaft 201, and an inflation lumen 206. Not shown, for the sake of clarity, is an external sheath that may optionally be used to encase balloon 202 and electrode array 203. A guiding element 204 such as a guidewire or balloon catheter may be used to guide device 200 to its target position. Guiding element 204 may have at its distal end an anchor 207 which may be a balloon, a shaped bend or a kink in guiding element 204, or any other expansion means or anchoring means as known in the art. RFG cable 208 connects electrodes 203 with the external RF generator RFG.

In use, in some embodiments, a colonoscopy may be performed prior to ablation. Device 200 may be inserted through the working channel of the colonoscope, which may typically have an inner diameter of ~3.7mm.

Since this lumen diameter is very small, it may be preferable to insert ablation device 200, which may have a larger diameter, in a different way. For example, the ablation device may be connected to a colonoscope, in a manner that enables it to be pulled alongside the colonoscope.

Alternatively, guiding element 204 may be inserted through the working channel of the colonoscope and left in place with anchor 207 at its tip positioned in the cecum or even inside the ileum. In some embodiments, guiding element 204 may be a balloon catheter and anchor 207 may be a balloon, which can be inflated to anchor it in place at the cecum or inside the distal ileum.

Ablation device 200 may be inserted over guiding element 204 by threading element 204 into guiding element lumen 205. Device 200 may be advanced until the distal opening of lumen 205 reaches anchor 207. At this position, location of the ileo-cecal valve and appendix relative to device 200 may be known, so they may be protected from ablation.

If an external sheath is used, it may be pulled back at this stage, and balloon 202 may be inflated until electrode array 203 is in contact with the intestinal wall. Energy may be delivered to perform the ablation, balloon may be deflated and pulled back, optionally into the external sheath.

The target depth of lesions created for treatment of the cecum and/or ascending colon may typically be 0.5-1mm.

Power settings for the ablation procedures described above may be:
For creating block of signal propagation:
Duration: typically 0.2-3seconds, preferably 0.5-2 seconds
Power: typically 5-50/watt/cm2, preferably 10-30/watt/cm2
Energy density: typically 1-20J/cm2, preferably 2-15J/cm2

For contracting tissue and reducing volume:
Duration: typically 0.5-20seconds, preferably 0.5-5 seconds
Power: typically 10-100/watt/cm2, preferably 20-50/watt/cm2
Energy density: typically 5-40J/cm2, preferably 10-30J/cm2

### Proportional ablation depending on the severity of the disease

The extent to which ablation of the colon is performed, in both length of colon treated, and depth of the lesions, may be adjusted according to the disease severity and/or patient characteristics, as detailed below.

In some embodiments, proportional ablation of progressively more colon may be performed, if needed. For example, some patients may do well with only a right colon ablation, but if necessary, for example if the response is insufficient, the treating physician may continue to ablate additional segments of the colon, and ablate the transverse colon, descending colon, sigmoid colon etc. For severely constipated patients the majority of the colon may need to be ablated. For elderly patients a right colon ablation may be sufficient.

In some embodiments, the choice of which layers of the colon to ablate, may be based on disease severity and anticipated response.

The mucosal layer of the colon is the surface layer that absorbs water. In some embodiments, ablation of the surface mucosal layer may reduce the capacity of the colon to absorb water, which may lead to more fluid in the colon and aid constipation. This modality of treatment may, in some embodiments, require ablation of large areas of mucosa, as opposed to just lines of ablation.

The deeper layers of the colon (muscle/submucosa) are mainly involved in contraction and scarring. In some embodiments, ablating the deeper layers of the colon may increase scarring and decrease distensibility of the colon, which may aid motility.

Thus, either superficial layer ablation for reducing fluid absorption, or deep layer ablation for inducing scarring and contraction of the colon, or both, may be performed as part of the treatment for constipation.

### Feedback regarding extent of therapy

In some embodiments, the process of gastric or colonic activity analysis may be repeated following treatment, to determine whether further treatment is required.

In some embodiments, sizing devices such as those described above, may be used during the ablation procedure, immediately after it, or several days or weeks after it, to assess the degree of change in organ size and/or compliance. In some embodiments, the sizing devices may alternatively or additionally be configured to calculate the change in compliance of the organ by measuring the change in pressure per unit volume inflated into the balloon while inside the organ. For example, in some embodiments, a sufficient treatment may be defined as a reduction of between 5% and 20% in the post-treatment vs pre-treatment measurements of the optimal device dimensions. In some embodiments, a sufficient treatment may be defined alternatively or additionally, as a similar reduction in organ compliance.

If treatment is deemed insufficient, the user may decide to repeat it.

### Data usage

Any measurements of gastrointestinal organ dimensions, compliance, impedance, activity, motility, or other parameters, obtained using the devices disclosed herein, as well as patient's clinical, demographic, procedural, and follow-up data, may optionally be used in various ways.

Typically, with the consent of the patients, such data may be gathered in a database, either locally, e.g. on a computer or local network, or on a distant or cloud based storage means.

In some embodiments, an algorithm may be provided, that may compare the current patient's parameters to the data in the database for supporting choice of device sizes and power settings, based on comparable historical procedure results.

For example, for treating a constipation patient, the algorithm may identify in the database previously treated patients with constipation, similar age, cecum size and cecum compliance, and recommend the device size and power settings that resulted in the best clinical outcome.

In some embodiments, additional patient information, such as results of skin elastography may be used to predict response to ablation and determine recommended power settings. While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that structures within the scope of these claims and their equivalents be covered thereby.

### List of Annotations:

| # | Name | Significance | Comments |
|---|---|---|---|
| 1 | elongate flexible bipolar electrode | | Embodiment demonstrating very general concept of linearly alternating poles |
| 2 | Core | | |
| 3 | conductor | | |
| 4 | conductor | | |
| 5 | contact | | Continuous with conductor 3 |
| 6 | contact | | Continuous with conductor 4 |
| | | | |
| 10 | elongate flexible spiral bipolar electrode | | main preferred embodiment - made of two wires twisted together |
| 11 | conductor | | |
| 12 | insulation layer | | |
| 13 | conductor | | |
| 14 | insulation layer | | |
| 15 | elongate ablation area | marks the area of tissue ablation or effect, which may result from delivery of energy to conductors 11 and 13 when in contact with a target tissue | |
| 16 | perimeter | marks the outer margins of the circle formed by the spiraled wires 11 and 13 | |
| a | | diameter of conductor 11 | |
| b | | thickness of insulation layer 12 | |
| c | | diameter of conductor 13 | |
| d | | thickness of insulation layer 14 | |
| g | gap | marks the gap between the insulation layers | |
| Dashed line A | | marks the outermost "peaks" of the spiraled wires before removal of insulation | |
| Dashed line B | | marks the extent to which insulation layers 12 and 14 may be removed | |
| C | | distance between lines A and B | |
| D | | marks the location of the cross section shown in FIG. 2D | passes through a "peak" and tissue contact point Q of conductor 11 |
| E | | marks the location of the cross section shown in FIG. 2E | passes through a "peak" and tissue contact point P of conductor 13 |
| F | | distance between lines D and E | |
| Q | Tissue contact point | | at line D |
| P | Tissue contact point | | at line E |
| W | Rectangular dashed box | marks the outermost area of electrode 10 where insulation layers 12 and 14 may be removed, as seen from top view | |
| R | Circular dashed line | denotes an area of removal of insulation and exposure of conductors | |
| 20 | elongate flexible spiral bipolar electrode | printed electrode | |
| 21 | flexible core | | |
| 22 | spiral conductor | | |
| 23 | spiral conductor | | |
| m | | distance between conductors 22 and 23 | |
| n | | distance between conductors 23 and 22 | |
| T | tissue | | |
| Abl | ablated (or affected) areas | | |
| 30 | elongate flexible spiral bipolar electrode | another printed or extruded electrode | |
| 31 | conductor | | |
| 32 | insulator core | | |
| 33 | conductor | | |
| 40 | jig | | |
| 41 | Jig shaft | | |
| 42 | Jig aperture | | |
| 43 | Distal attachment | | |
| 44 | Proximal attachment | | |
| 45 | Tension means | | |
| 101 | Stomach | | |
| 102 | Cardia | | |
| 103 | Esophagogastric junction | | |
| 104 | Fundus | | |
| 105 | Antrum | | |
| 106 | Pylorus | | |
| 107 | Lesser curve | | |
| 108 | Greater curve | | |
| 109 | Angularis | | |
| COR | Corpus | | |
| MCS | Mucosa | | |
| SMC | Submucosa | | |
| MUS | Muscularis | | |
| SER | Serosa | | |
| 110 | Gastric wall | | |
| 111 | Gastric glands | | |
| 112 | Muscularis mucosae | | |
| 113 | Oblique muscle layer | | |
| 114 | Circular muscle layer | | |
| 115 | Longitudinal muscle layer | | |
| 116 | Visceral peritoneum | | |
| SP | Submucosal Plexus | | |
| MP | Myenteric Plexus | | |
| 120A | Global spiral ablation pattern | | |
| 120B | Global linear axial ablation pattern | | |
| 120C | Global linear circumferential ablation pattern | | |
| 120D | Global grid ablation pattern | | |
| 120E | Global axial midline ablation pattern | | |
| 120F | Global circumferential midline ablation pattern | | |
| 120G | Circumferential "constriction ring" ablation pattern | | |
| 120H | Segmented circumferential "constriction ring" ablation pattern | | |
| 130A | Regional spiral ablation pattern | | |
| 130B | Regional linear axial ablation pattern | | |
| 130C | Regional linear circumferential ablation pattern | | |
| 130D | Regional grid | | |
| | ablation pattern | | |
| 140 A,B,C | Local ablation pattern x + circle | | |
| 141 A,B,C | Local ablation pattern x | | |
| 142 A,B,C | Local ablation pattern spiral | | |
| 143 | Combined ablation pattern (global circumferential midline and local x + circle) | | |
| 144 | Outline of gastric wall around untreated fundus | | |
| 144' | Outline of gastric wall around treated fundus | | |
| 145 | Ablation pattern for shrinkage of fundus | | |
| 150A | Global pattern ablation device | | Shaft goes into duodenum, single balloon |
| 151 | Handle | | |
| 152 | Shaft | | |
| 153 | Expandable member / balloon | | |
| 154 | Sheath | | |
| 155 | Expansion port and lumen | | |
| 156 | Openings | | |
| 157 | Guidewire lumen | | |
| 158 | Guidewire | | |
| 159 | Sheath port | | |
| 161 | Conductors | | |
| 162 | Cable | | |
| 163 | Tip | | |
| 164 | Seal | | |
| RFG | | | |
| 150B | Global pattern ablation device | | As in 150A, two balloons |
| 153' | Smaller balloon | | |
| 153'' | Additional balloon | | Fills fundus |
| 150C | Global pattern ablation device | | Single balloon, shaft ends at pylorus |
| 160 | Regional pattern ablation device | | |
| 170A | Local pattern ablation device | | |
| 171 | Conductors | | |
| 172 | Balloon | | |
| 173 | Rigid shaft | | |
| 174 | Sheath | | |
| 173' | Curved rigid shaft | | |
| 174' | Curved sheath | | |
| 180 | large intestine | | |
| 181 | cecum | | |
| 182 | ascending (right) colon | | |
| 183 | transverse colon | | |
| 184 | descending (left) colon | | |
| 185 | sigmoid colon | | |
| 186 | rectum | | |
| 187 | dilated cecum and ascending colon | | |
| 190A | Spiral ablation pattern in cecum and ascending colon | | |
| 190B | Interlacing half circles ablation pattern in cecum and ascending colon | | |
| 200 | Cecum and/or ascending colon ablation device | | |
| 201 | Flexible shaft | | |
| 202 | Balloon | | |
| 203 | Electrode array | | |
| 204 | Guiding element | | |
| 205 | Guiding element lumen | | |
| 206 | Inflation lumen | | |
| 207 | anchor | | |
| 208 | RFG cable | | |
| ILE | Distal ileum | | |
| 300 | Tissue ablation | | |
| | device with combined spiral flexible bipolar electrodes and rail track electrodes | | |
| 302 | Flexible spiral bipolar electrodes | | |
| 304 | Inner shaft | | |
| 306 | External sheath | | |
| 308 | Parallel "rail track" electrodes | | |
| 310 | Longitudinal PCB legs | | |
| 312 | Distal tip | | |
| 314 | Expandable element | | |

## Claims

1. A device (300) for treating a medical condition, the device (300) comprising at least one deployable ablation element (302, 308, 310), an expandable member (314), a shaft (304), and
wherein the device has a crimped position, a deployed position, and a fully expanded position, and
wherein the at least one ablation element (302, 308, 310) is positioned over the expandable member (314), and the expandable member (314) is mounted over the shaft (304), and
wherein in the crimped state the device is configured for insertion into a hollow organ of a patient, and
wherein in the fully expanded position the at least one ablation element (302, 308, 310) is configured to come into contact with the wall of the hollow organ.

2. The device of claim 1 wherein the hollow organ is any of a stomach, a duodenum, a small bowel, a large bowel, a cecum, or an ascending colon.

3. The devices of claims 1 or 2 wherein the at least one ablation element (302, 308, 310) is a conductor.

4. The devices of claims 1 to 3 wherein the conductor is a bipolar spiral electrode (302).

5. The devices of claims 1 to 4 wherein the expandable member is a balloon.

## Patentansprüche

1. Vorrichtung (300) zur Behandlung eines medizinischen Leidens, wobei die Vorrichtung (300) mindestens ein entfaltbares Ablationselement (302, 308, 310), ein ausdehnbares Element (314), einen Schaft (304) umfasst, und
wobei die Vorrichtung eine gefaltete Position, eine entfaltete Position und eine vollständig ausgedehnte Position aufweist, und
wobei das mindestens eine Ablationselement (302, 308, 310) über dem ausdehnbaren Element (314) positioniert ist und das ausdehnbare Element (314) über dem Schaft (304) montiert ist, und
wobei die Vorrichtung in dem gefalteten Zustand zur Einführung in ein Hohlorgan eines Patienten konfiguriert ist, und
wobei das mindestens eine Ablationselement (302, 308, 310) in der vollständig ausgedehnten Position dazu konfiguriert ist, in Kontakt mit der Wand des Hohlorgans zu kommen.

2. Vorrichtung nach Anspruch 1, wobei das Hohlorgan ein beliebiges von einem Magen, einem Zwölffingerdarm, einem Dünndarm, einem Dickdarm, einem Blinddarm oder einem Colon ascendens ist.

3. Vorrichtungen nach den Ansprüchen 1 oder 2, wobei das mindestens eine Ablationselement (302, 308, 310) ein Leiter ist.

4. Vorrichtungen nach den Ansprüchen 1 bis 3, wobei der Leiter eine bipolare Spiralelektrode (302) ist.

5. Vorrichtungen nach den Ansprüchen 1 bis 4, wobei das ausdehnbare Element ein Ballon ist.

## Revendications

1. Dispositif (300) destiné à traiter une affection médicale, le dispositif (300) comprenant au moins un élément d'ablation capable de déploiement (302, 308, 310), un élément capable de développement (314), un arbre (304), et
dans lequel le dispositif a une position sertie, une position déployée, et une position entièrement développée, et
dans lequel l'au moins un élément d'ablation (302, 308, 310) est positionné sur l'élément capable de développement (314), et l'élément capable de développement (314) est monté sur l'arbre (304), et
dans lequel dans l'état serti le dispositif est configuré pour être inséré jusque dans un organe creux d'un patient, et
dans lequel dans la position entièrement développée l'au moins un élément d'ablation (302, 308, 310) est configuré pour entrer en contact avec la paroi de l'organe creux.

2. Dispositif selon la revendication 1 dans lequel le corps creux est l'un quelconque d'un estomac, d'un duodénum, d'un intestin grêle, d'un gros intestin, d'un cécum, ou d'un côlon ascendant.

3. Dispositifs selon les revendications 1 ou 2 dans lesquels l'au moins un élément d'ablation (302, 308, 310) est un conducteur.

4. Dispositifs selon les revendications 1 à 3 dans lesquels le conducteur est une électrode en spirale bipolaire (302).

5. Dispositifs selon les revendications 1 à 4 dans lesquels l'élément capable de développement est un ballonnet.
